# EUROPEAN PATENT APPLICATION

(11) **EP 2 025 312 A1**
(43) Date of publication of application: **18.02.2009**
(21) Application number: 07743975.0
(22) Date of filing: 23.05.2007
(51) Int. Cl.: A61F 13/15, A61F 13/514, A61F 13/56

(54) **ABSORBENT ARTICLE**

(30) Priority: 02.06.2006 JP 2006155113; 09.03.2007 JP 2007060995
(71) Applicant: UNI-CHARM CORPORATION, Shikokuchuo-shi, Ehime 799-0111 (JP)
(72) Inventor: NODA, Yuki, Kanonji-shi, Kagawa 769-1602 (JP); KURODA, Kenichiro, Kanonji-shi, Kagawa 769-1602 (JP)
(74) Representative: Fitchett, Stuart Paul
(86) International application number: PCT/JP2007/060541
(87) International publication number: WO 2007/142036

(57) **Abstract**

An absorbent article (1) is provided with a body (5) of the absorbent article having at least a top sheet portion (2), which is liquid-permeable at least in a part thereof and located in one side in the direction thickness of the absorbent article, and a liquid retainable absorbent portion (4), which is located in the other side of the top sheet portion (2) in the direction thickness as described above, and a belt-shaped member (10) which is located in the abovedescribed other side in the direction thickness of the body (5) of the absorbent article (1) along the longitudinal direction LD (MD) of the body (5), wherein one end side of the belt-shaped member (10) in the longitudinal direction LD (MD) is bonded to the body (5) of the absorbent article (1).

## Description

### TECHNICAL FIELD

The present invention relates to an absorbent article.

### BACKGROUND ART

As an absorbent article for absorbing excrement such as menstrual blood, sanitary napkins, panty liners, urine-absorbing pads, and the like have been used conventionally. These absorbent articles have an absorbent body that forms an absorption layer for absorbing and holding menstrual blood or the like, a liquid permeable top sheet that covers the surface on a skin contacting surface of the absorbent body, and a liquid impermeable back sheet that covers the back positioned on the clothing side of the absorbent body. For example, these absorbent articles can be used in a state where these are adhered to the internal surface of a groin contacting portion of underwear.

In order to catch excrement such as menstrual blood, it is desired that the abovementioned absorbent articles be used in a state where the absorption layer having the absorbent body is adhered to the excretory part of a wearer. However, since the absorbent articles are by nature adapted for use in the state where these are attached to underwear or the like, both are susceptible to relative dislocation between the underwear and the excretory part. If there is a space between the excretory part and the absorbent article, the excrement dropped on the top sheet may effuse along the top sheet toward the sides and the buttocks, resulting in the soiling of the underwear and clothing.

In this connection, for example, Japanese Utility Model Application Laid-Open No. Hei 3-101933 (hereinafter referred to as "patent document 1") discloses an absorbent article having improvements in adhesion (adhesive properties) of the absorbent article to the human body during the time underwear is put on. Specifically, flexible flaps are formed on both ends in the longitudinal direction of the absorbent article, and retainers provided at the flaps can be adhered to the underwear.

On the other hand, Japanese Patent Application Laid-Open No. Hei 11-99179 (hereinafter referred to as "patent document 2") discloses an absorbent article having improvements in adhesion of the absorbent article to the human body when underwear is put on. Specifically, a flexible elastic member can be extended from the edge portion of a sanitary napkin, and an adhesive region is formed on a contact surface with the underwear located at the portion so extended. The adhesive region is then adhered to the underwear or the like.

That is, each of the absorbent articles as disclosed in patent document 1 and patent document 2 is provided with the elastic portions on both ends of the absorbent article in the longitudinal direction, and adapted to improve adhesion by pulling the absorbent article toward the excretory part of the wearer by the elastic force generated in the elastic members.

### DISCLOSURE OF THE INVENTION

### Problems to be Solved by the Invention

However, the absorbent articles disclosed in patent document 1 and patent document 2 have the configuration to pull the absorbent article in front and behind by the elastic force of the elastic portions on both ends of the absorbent article. Hence, both might be unable to obtain sufficient force that the absorbent article is adhered to the excretory part.

The present invention has been made in view of the foregoing problem, and aims at providing an absorbent article that prevents the leakage of excrement, such as menstrual blood, by improving the adhesion between the excretory part or the like of the wearer and the absorbent article.

### Means for Solving the Problems

The present inventors, for the abovementioned purpose, have achieved the present invention based on the discovery that the absorbent article body can be adhered to the excretory part or the like by providing a belt-shaped member, one end of which is fixed to an absorbent article body, on a non-skin contact surface of the absorbent article body, and by fixing the belt-shaped member to underwear or the like with the entire absorbent article body lifted. Specifically, the present invention provides the following absorbent articles.

In a first aspect of an absorbent article including an absorbent article body having at least: a top sheet portion disposed on a first side of the absorbent article having a substantially elongated shape in a thickness direction, at least a portion of the top sheet portion being liquid permeable, and a liquid retainable absorbent body portion disposed on a second side of the top sheet portion, the absorbent body portion being disposed on a second side of the absorbent article in the thickness direction; and a belt-shaped member disposed along a longitudinal direction of the absorbent article body, the belt-shaped member being disposed on the second side of the absorbent article body. One end of the belt-shaped member in the longitudinal direction is connected to the absorbent article body.

According to the first aspect of the present invention, the absorbent article includes the absorbent article body having: the top sheet portion disposed on a first side of the absorbent article in the thickness direction, at least a portion of the top sheet portion being liquid permeable, and the liquid retainable absorbent body portion disposed on the second side of the top sheet portion, the liquid retainable absorbent body portion being disposed on the second side of the absorbent article in the thickness direction; and the belt-shaped member disposed along the longitudinal direction of the absorbent article body, the belt-shaped member being disposed on a second side of the absorbent article body. The belt-shaped member is connected, on the longitudinal direction thereof, to the absorbent article body.

Thus, in the above absorbent article, the belt-shaped member, a first end of which is fixed to the absorbent article body and a second end is a free end, is disposed on the second end of the absorbent article body. The free end is then fixed to or engaged with a predetermined location of an object to be engaged (hereinafter referred to as an engaged object) such as underwear, thereby enabling the entire absorbent article body to be lifted to the human body. This improves the adhesion between the wearer's excretory part or the like and the absorbent article, enabling the prevention of leakage of excrement such as menstrual blood.

The term "lifted to the human body" includes the state in which the non-skin contact surface of the absorbent article body, the belt-shaped member disposed along the longitudinal direction of the absorbent article body lifts the absorbent article body in cooperation with a fixing portion to be fixed with the absorbent article body, and an engaging portion to be engaged with the engaged object.

In a second aspect of the absorbent article as described in the first aspect, at least a portion of the belt-shaped member is formed so as to be extensible in the longitudinal direction.

According to the second aspect of the present invention, at least a portion of the belt-shaped member is formed so as to be extensible in the longitudinal direction. The extensibility of the belt-shaped member in the longitudinal direction can ensure a predetermined length of the belt-shaped member required when it is attached to underwear as an engaged object. Additionally, since the belt-shaped member is not extended when it is not attached thereto, it is possible to reduce the size of the belt-shaped member in the absorbent article.

The term "formed so as to be extensible" means, for example, applying a corrugated embossing finish to or forming a predetermined flap in the belt-shaped member. Alternatively, an extensible base material sheet may be used.

In a third aspect of the absorbent article as described in the first aspect, at least a portion of the belt-shaped member is formed so as to be able to contract in the longitudinal direction.

According to the third aspect of the present invention, at least a portion of the belt-shaped member is formed so as to be flexible, able to contract in the longitudinal direction. The flexibility of the belt-shaped member in the longitudinal direction enables the state where the absorbent article body is adhered to the human body to be maintained. This is because, for example, even if a relative slip occurs between the absorbent article body and the human body due to the loosening of underwear or deformation from the movement of the human body, the belt-shaped member can expand and contract according to the slip. It is therefore possible to improve the adhesion between the wearer's excretory part or the like and the absorbent article, enabling the prevention of leakage of excrement such as menstrual blood.

In a fourth aspect of the absorbent article as described in any one of the first to third aspects, a cover portion disposed on the second side of the absorbent article body is further included, the cover portion covering at least a portion of the belt-shaped member in the longitudinal direction and covering the entire of the belt-shaped member in a width direction.

According to the fourth aspect of the present invention, the absorbent article has, on the second side in the thickness direction, the cover portion covering the belt-shaped member. The cover portion covers at least a portion of the belt-shaped member in the longitudinal direction and covers the entire of the belt-shaped member in the width direction. That is, the cover portion is disposed so as to cover the width direction of the absorbent article on the second side of the absorbent article. The belt-shaped member is disposed between the absorbent article and the cover portion. At this time, the belt-shaped member is arranged so as to be slidable in between the absorbent article body and the cover portion. Then, the belt-shaped member can be extended from an opening formed between the absorbent article body and the cover portion on one end or both ends in the longitudinal direction. Therefore, the cover portion covering the belt-shaped member enables the absorbent article to keep the belt-shaped member clean. The cover portion is also capable of guiding the direction in which the belt-shaped member slides. In an alternative, the cover portion may cover a portion or the entire of the longitudinal direction of the belt-shaped member. In another alternative, a plurality of the cover portions may be provided in the longitudinal direction.

In a fifth aspect of the absorbent article as described in any one of the first to fourth aspects, the belt-shaped member has a grip portion on the other end in the longitudinal direction, the grip portion extending from an outer edge of the absorbent article body in the longitudinal direction.

According to the fifth aspect of the present invention, the belt-shaped member has the grip portion extending from the outer edge of the absorbent article body, on a second end in the longitudinal direction. The grip portion extending from the outer edge of the absorbent article body facilitates confirmation of the position of the belt-shaped member at the time of attachment. The presence of the grip portion can facilitate the attachment, for example.

In a sixth aspect of the absorbent article as described in the fifth aspect, the grip portion has a guide element indicating a direction of extension of the belt-shaped member.

According to the sixth aspect of the present invention, the grip portion has the guide element indicating a direction of extension of the belt-shaped member. For example, the guide element can guide the belt-shaped member in a predetermined direction at the time of attachment. This enables the wearer to confirm a suitable manner of attachment by confirming the guide element. Even if it is not possible to confirm by visual observation, the guide member can be confirmed by tactile sense or the like, thereby preventing misattachment.

In a seventh aspect of the absorbent article as described in the fifth or sixth aspect, the second end of the belt-shaped member is provided with a predetermined engaging portion.

According to the seventh aspect of the present invention, the second end of the belt-shaped member is provided with the predetermined engaging portion. This enables the engaging portion to be engaged to a predetermined location of an engaged object such as underwear. This improves the adhesion between the wearer's excretory part or the like and the absorbent article, enabling the prevention of leakage of excrement such as menstrual blood.

In an eighth aspect of the absorbent article as described in any one of the fifth to seventh aspects, the second end of the belt-shaped member is provided with a predetermined absorptive member.

According to the eighth aspect of the present invention, the second end of the belt-shaped member is provided with the predetermined absorptive member. Thus, the grip portion can increase the strength by having the absorptive member. This can prevent the grip portion from being deformed when the grip portion is pulled to engage the engaging portion with a predetermined location of an engaged object such as underwear. The prevention of deformation of the grip portion enables the prevention of, for example, the deformation of the engaging portion disposed on the other end of the belt-shaped member, thus facilitating engagement of the engaging portion with the engaged object.

In a ninth aspect of the absorbent article as described in the eighth aspect, the absorptive member is disposed in a region extending from the outer edge of the absorbent article body in the longitudinal direction when the belt-shaped member extends, or it expands and contracts.

According to the ninth aspect of the present invention, the absorptive member is disposed in the region extending from the outer edge of the absorbent article body in the longitudinal direction when the belt-shaped member extends, or it expands and contracts. Thus, the belt-shaped member can also absorb, for example, excrement such as body fluid leaked from the absorbent article body and the excrement effused along the wearer's body when attaching the absorbent article.

In a tenth aspect of the absorbent article as described in the eighth or ninth aspect, the belt-shaped member has a liquid impermeable sheet disposed in a region corresponding to the absorptive member on the opposite side of the absorbent article body.

According to the tenth aspect of the present invention, the belt-shaped member has the liquid impermeable sheet disposed in the region corresponding to the absorptive member on the opposite side of the absorbent article body. This enables prevention of the excrement such as bodily fluid absorbed by the absorptive member of the belt-shaped member from soaking through an engaged object such as underwear.

In an alternative, the liquid impermeable sheet may be arranged so as to cover the outer edge on the second end of the belt-shaped member. This enables prevention of the absorbed excrement or the like from soaking through the outer edge of the belt-shaped member. In another alternative, the liquid impermeable member may be arranged so as to cover at least a portion of both side portions in the region extending from the outer edge of the absorbent article body in the longitudinal direction. This enables prevention of the absorbed excrement or the like from soaking through both side portions of the belt-shaped member.

In an eleventh aspect of the absorbent article as described in any one of the first to tenth aspects, a length of the belt-shaped member in the width direction is in a range of at least 30% of a length of the absorbent article body in the width direction.

According to the eleventh aspect of the present invention, the length of the belt-shaped member in the width direction is in the range of at least 30% of the length of the absorbent article body in the width direction. Thus, the belt-shaped member can be brought into contact with a predetermined range of the absorbent article body in the width direction. As a result, for example, the entire absorbent article body can be lifted with the suitable form thereof retained. This enables the absorbent article body to be adhered to a predetermined portion of the wearer's body, thereby preventing the leakage of excrement such as menstrual blood.

In a twelfth aspect of the absorbent article as described in any one of the first to eleventh aspects, the belt-shaped member has a belt-shaped base material sheet and an elastic member.

According to the twelfth aspect of the present invention, the belt-shaped member has the belt-shaped base material sheet and the elastic member. This enables the prevention of so-called neck in, which could be caused when a belt-shaped elastic member is used, namely the phenomenon that the substantially central portion thereof becomes narrow.

In a thirteenth aspect of the absorbent article as described in any one of the first to twelfth aspects, at least a portion of the belt-shaped member has a liquid impermeable material.

According to the thirteenth aspect of the present invention, at least a portion of the belt-shaped member has the liquid impermeable material. Thus, without a liquid impermeable back sheet portion used in the absorbent article body, it is possible to prevent leakage to the other side of the absorbent article by disposing the belt-shaped member on the other side of the absorbent body portion.

In a fourteenth aspect, an absorbent article includes an absorbent article body having at least: a top sheet portion, at least a portion of the top sheet portion being liquid permeable, the top sheet portion being disposed on a first side of the absorbent article having a substantially elongated shape in a thickness direction, the absorbent article being used by bringing it into contact with a human body in a state of being disposed on an internal surface of a predetermined clothing, and a liquid retainable absorbent body portion disposed on a second side of the top sheet portion, the absorbent body portion being disposed on a second side of the absorbent article in the thickness direction; and a belt-shaped member disposed along a longitudinal direction of the absorbent article body, the belt-shaped member being disposed on the second side of the absorbent article body. The belt-shaped member can be disposed along the human body by using the belt-shaped member in a state where it is extended and engaged with an engaged object.

According to the fourteenth aspect of the present invention, the absorbent article includes the absorbent article body having: the top sheet portion disposed on a first end of the absorbent article in the thickness direction, at least a portion of the top sheet portion being liquid permeable, and the liquid retainable absorbent body portion disposed on the second side of the top sheet portion, the absorbent body portion being disposed on the second side of the absorbent article in the thickness direction; and the belt-shaped member disposed along the longitudinal direction of the absorbent article body, the belt-shaped member being disposed on the second side of the absorbent article body. The belt-shaped member is used in the state where it is extended and engaged with, for example, the internal surface of predetermined clothing such as underwear.

This enables the belt-shaped member to press the absorbent article body upwards along a predetermined portion of the wearer. For example, it is possible to attain the state of lifting along the curvature of the wearer's body. Furthermore, the force generated in the belt-shaped member can be directed from the entire absorbent article body to a predetermined part of the wearer. As a result, the entire of the absorbent article body can be adhered to the predetermined part of the wearer.

In a fifteenth aspect, an absorbent article including an absorbent article body having at least: a top sheet portion, at least a portion of the top sheet portion being liquid permeable, the top sheet portion being disposed on a first side of the absorbent article in a thickness direction, the absorbent article having a first manner of application and a second manner of application, and a liquid retainable absorbent body portion disposed on a second side of the top sheet portion, the absorbent body portion being disposed on a second side of the absorbent article in the thickness direction; and a belt-shaped member disposed along a longitudinal direction of the absorbent article body, the belt-shaped member being disposed on the second side of the absorbent article body. The first manner of application is using the absorbent article by engaging the belt-shaped member with an engaged object. The second manner of application is using the absorbent article by not engaging the belt-shaped member with the engaged object.

According to the fifteenth aspect of the present invention, the absorbent article includes the absorbent article body having: the top sheet portion disposed on a first side in the thickness direction, at least a portion of the top sheet portion being liquid permeable, and the liquid retainable absorbent body portion disposed on the second side of the top sheet portion, the liquid retainable absorbent body portion being disposed on a second side of the absorbent article in the thickness direction; and the belt-shaped member disposed along the longitudinal direction of the absorbent article body, the belt-shaped member being disposed between the top sheet portion and the absorbent body portion. The absorbent article has the first manner of application for using it by engaging the belt-shaped member with an engaged object, for example, underwear or the wearer's body, and the second manner of application for using it by not engaging the belt-shaped member with the engaged object, for example, underwear or the wearer's body.

Thus, the absorbent article may be used by engaging the belt-shaped member with predetermined underwear or the like, or alternatively, by not engaging it therewith. This enables the wearer to select the manner of application depending on the wearer's situation.

In a sixteenth aspect of the absorbent article as described in the fifteenth aspect, the first manner of application is for arranging the belt-shaped member along the human body in a state where the belt-shaped member is extended and engaged with the predetermined engaged object.

According to the sixteenth aspect of the present invention, in the first manner of application in the absorbent article, the belt-shaped member is used in the state where it is extended and engaged with a predetermined engaged object such as underwear or the wearer's body. This manner of application enables the belt-shaped member to press the absorbent article body against a predetermined part of the wearer. For example, it is possible to attain the state of pressing against the curvature of the wearer's body. Furthermore, the force generated in the belt-shaped member can be directed from the entire of the absorbent article body to a predetermined part of the wearer, enabling the entire absorbent article body to be in contact with the predetermined part of the wearer.

In a seventeenth aspect, the absorbent article including an absorbent article body having at least: a top sheet portion disposed on a first side of the absorbent article having a substantially elongated shape in a thickness direction, at least a portion of the top sheet portion being liquid permeable, and a liquid retainable absorbent body portion disposed on a second side of the top sheet portion, the absorbent body portion being disposed on a second side of the absorbent article in the thickness direction; and a liquid permeable belt-shaped member disposed between the top sheet portion and the absorbent body portion so as to be slidable along a longitudinal direction of the absorbent article body. One end of the belt-shaped member in the longitudinal direction is connected to the absorbent article body.

According to a seventeenth aspect of the present invention, the absorbent article includes the absorbent article body having: the top sheet portion disposed on a first side of the absorbent article in the thickness direction, at least a portion of the top sheet portion being liquid permeable, and the liquid retainable absorbent body portion disposed on the second side of the top sheet portion, the absorbent body portion being disposed on a second side of the absorbent article in the thickness direction; and the liquid permeable belt-shaped member disposed between the top sheet portion and the absorbent body portion so as to be slidable along the longitudinal direction of the absorbent article body. The absorbent article is formed in a substantially elongated shape. The belt-shaped member is connected to the absorbent article body at one end of the belt-shaped member in the longitudinal direction.

Thus, the belt-shaped member, one end of which is a free end, is disposed so as to be slidable in between the top sheet portion and the absorbent body portion in the absorbent article body, and the free end is fixed to a predetermined location of an engaged object such as underwear, so that the material closer to the skin surface than the absorbent body portion, namely the top sheet, can be lifted to the human body by the belt-shaped member. Therefore, even a wearer with sensitive skin is not always subjected to the stiffness of the absorbent body portion. It is also possible to prevent the leakage of menstrual blood effused along the skin.

In an eighteenth aspect of the absorbent article as described in the seventeenth aspect, at least a portion of the belt-shaped member is formed so as to be extensible in the longitudinal direction.

According to the eighteenth aspect of the present invention, at least a portion of the belt-shaped member is formed so as to be extensible. The extensibility of the belt-shaped member can ensure, for example, a predetermined length of the belt-shaped member necessary for the attachment to underwear as an engaged object. Additionally, since the belt-shaped member is not extended when it is not attached thereto, it is possible to reduce the size of the belt-shaped member in the absorbent article.

In a nineteenth aspect of the absorbent article as described in the seventeenth aspect, at least a portion of the belt-shaped member is formed so as to be flexible in the longitudinal direction.

According to the nineteenth aspect of the present invention, at least a portion of the belt-shaped member is formed so as to be flexible. The flexibility of the belt-shaped member enables the state where the absorbent article body is contacted to the human body to be maintained. This is because, for example, even if a relative slip occurs between the absorbent article body and the human body due to the loosening of underwear or deformation from movement of the human body, the belt-shaped member can expand and contract according to the slip. Hence, by improving the contact between the wearer's excretory part or the like and the absorbent article, it is possible to prevent the leakage of excrement such as menstrual blood.

### Effects of the Invention

Accordingly, the present invention can provide absorbent articles capable of preventing the leakage of excrement, such as menstrual blood, by improving adhesion with the excretory part or the like of the wearer.

### BRIEF DESCRIPTION OF THE DRAWINGS

Fig. 1 is a front view of a sanitary napkin according to a first preferred embodiment of the present invention;
Fig. 2 is a back view of Fig. 1;
Fig. 3 is a sectional view taken along the line A-A of Fig. 1;
Fig. 4A is a sectional view taken along the line B-B of Fig. 1;
Fig. 4B is a sectional view taken along the line C-C of Fig. 1;
Fig. 4C is a sectional view taken along the line D-D of Fig. 1;
Fig. 5 is a front view of a belt-shaped member according to the first preferred embodiment;
Fig. 6 is a front view showing another embodiment of the belt-shaped member;
Fig. 7A is a sectional view taken along the line E-E of Fig. 6;
Fig. 7B is a sectional view taken along the line F-F of Fig. 6;
Fig. 7C is a sectional view taken along the line G-G of Fig. 6;
Fig. 8 is a diagram showing still another embodiment of the belt-shaped member;
Fig. 9A is a partially enlarged view of Fig. 8;
Fig. 9B is a partially enlarged view showing yet another embodiment of the belt-shaped member;
Fig. 10 is a front view showing the configuration when attaching the sanitary napkin according to the first preferred embodiment;
Fig. 11A is a diagram showing the attached state of the sanitary napkin according to the first preferred embodiment;
Fig. 11B is a diagram showing the attached state of the sanitary napkin according to the first preferred embodiment;
Fig. 11C is a diagram showing the attached state of the sanitary napkin according to the first preferred embodiment;
Fig. 12 is a front view of a sanitary napkin according to a second preferred embodiment of the present invention;
Fig. 13 is a back view of Fig. 12;
Fig. 14 is a sectional view taken along the line H-H of Fig. 12;
Fig. 15A is a sectional view taken along the line I-I of Fig. 12;
Fig. 15B is a sectional view taken along the line J-J of Fig. 12;
Fig. 15C is a sectional view taken along the line K-K of Fig. 12;
Fig. 16 is a sectional view taken along the line H-H, showing a modification of the sanitary napkin according to the second preferred embodiment;
Fig. 17 is a front view of a sanitary napkin according to a third preferred embodiment of the present invention;
Fig. 18 is a sectional view taken along the line L-L of Fig. 17;
Fig. 19A is a sectional view taken along the line M-M of Fig. 17;
Fig. 19B is a sectional view taken along the line N-N of Fig. 17;
Fig. 19C is a sectional view taken along the line O-O of Fig. 17;
Fig. 20 is a front view of a sanitary napkin according to a fourth preferred embodiment of the present invention;
Fig. 21A is a sectional view taken along the line P-P according to another embodiment of Fig. 20;
Fig. 21B is a sectional view taken along the line P-P of Fig. 20;
Fig. 22A is a front view of a sanitary napkin according to a fifth preferred embodiment of the present invention;
Fig. 22B is a sectional view taken along the line Q-Q of Fig. 22A;
Fig. 23 is a front view of a sanitary napkin according to a sixth preferred embodiment of the present invention;
Fig. 24A is a sectional view taken along the line R-R of Fig. 23;
Fig. 24B is a sectional view taken along the line S-S of Fig. 24A;
Fig. 25 is a front view of a sanitary napkin according to a seventh preferred embodiment of the present invention;
Fig. 26A is a sectional view taken along the line T-T of Fig. 25;
Fig. 26B is a diagram showing a modification of the cross section taken along the line T-T of Fig. 25;
Fig. 27A is a front view of a sanitary napkin according to an eighth preferred embodiment of the present invention;
Fig. 27B is a front view of a sanitary napkin according to the eighth preferred embodiment of the present invention;
Fig. 28A is a front view of a sanitary napkin according to a ninth preferred embodiment of the present invention;
Fig. 28B is a front view of a sanitary napkin according to the ninth preferred embodiment of the present invention;
Fig. 29 is a front view of a sanitary napkin according to a tenth preferred embodiment of the present invention;
Fig. 30A is a front view of a sanitary napkin according to an eleventh preferred embodiment of the present invention;
Fig. 30B is a front view of a sanitary napkin according to an eleventh preferred embodiment of the present invention;
Fig. 31A is a front view of a sanitary napkin according to a twelfth preferred embodiment of the present invention;
Fig. 31B is a sectional view taken along the line U-U of Fig. 31A;
Fig. 32 is a front view of a sanitary napkin according to a thirteenth preferred embodiment of the present invention;
Fig. 33 is a front view of a sanitary napkin according to a fourteenth preferred embodiment of the present invention;
Fig. 34 is a front view of a sanitary napkin according to a fifteenth preferred embodiment of the present invention;
Fig. 35 is a front view of a sanitary napkin according to a sixteenth preferred embodiment of the present invention; and
Fig. 36 is a sectional view taken along the line V-V of Fig. 35.

### PREFERRED MODE FOR CARRYING OUT THE INVENTION

Preferred embodiments of the present invention are described below with reference to the accompanying drawings. However, it is to be understood that the embodiments of the present invention are not limited to the following examples, and the technical scope of the present invention is not limited to these.

Although the absorbent articles according to the present invention are positioned on the crotch of the human body in order to absorb menstrual blood, urine, and leukorrhea discharged from the human body, the following preferred embodiments are directed to sanitary napkins, the primary object of which is to absorb menstrual blood discharged from the vaginal opening of females. In the following description, one of the two surfaces of the absorbent article which is directed to the excretory part is called "skin contact surface," and the other is called "non-skin contact surface" irrespective of whether or not clothing is worn on the outside thereof.

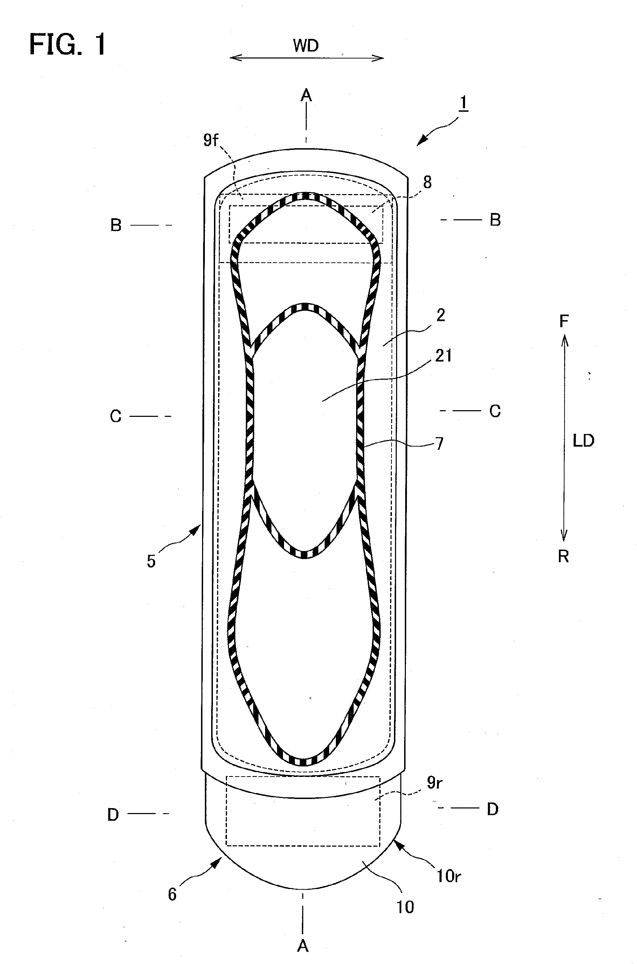

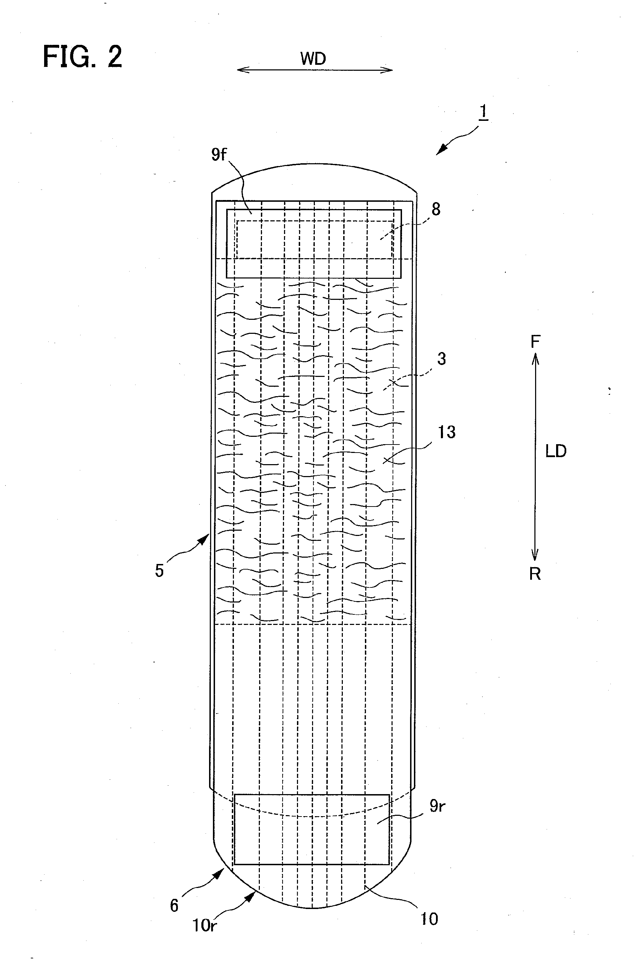

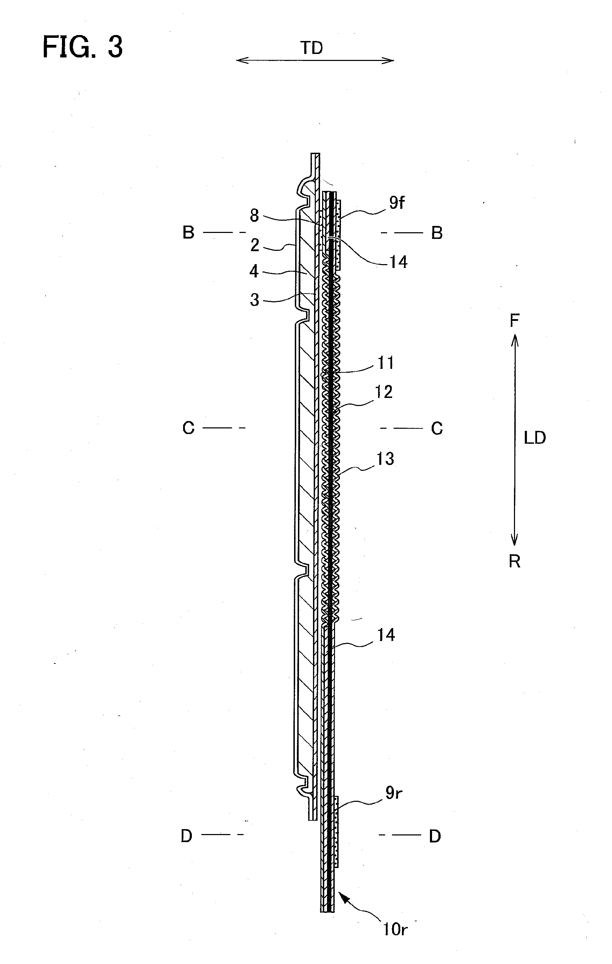

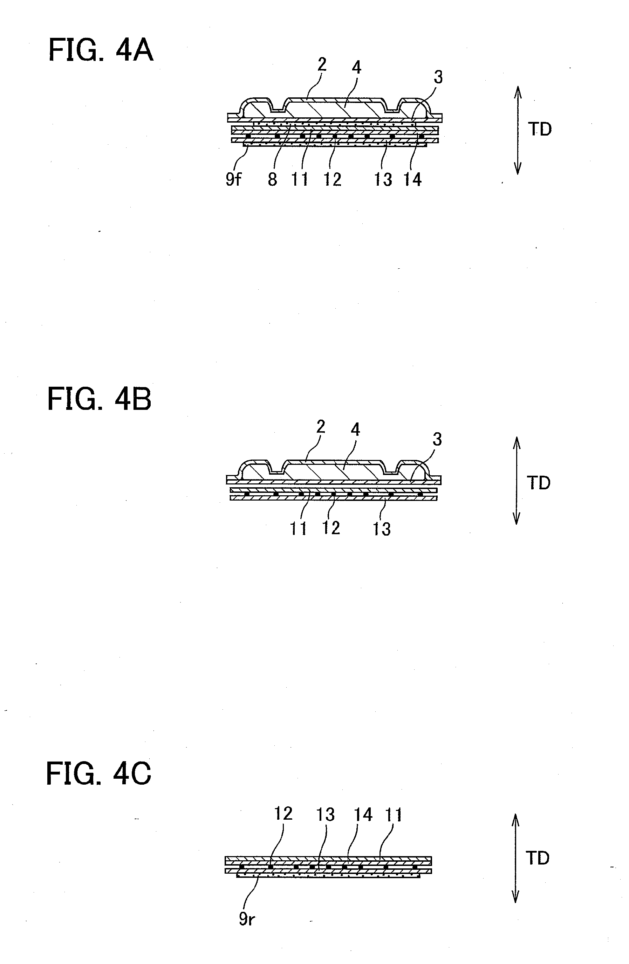

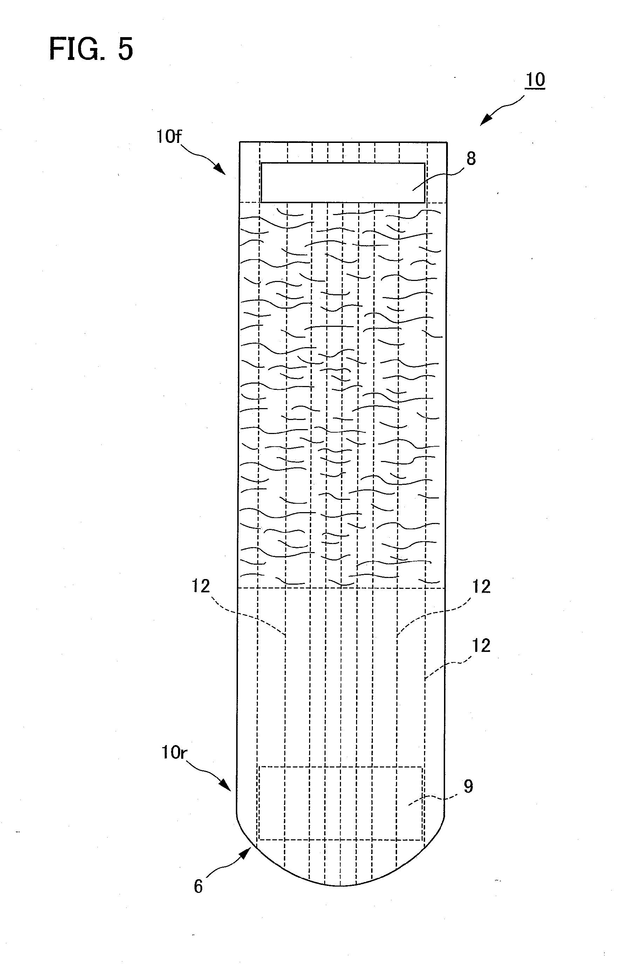

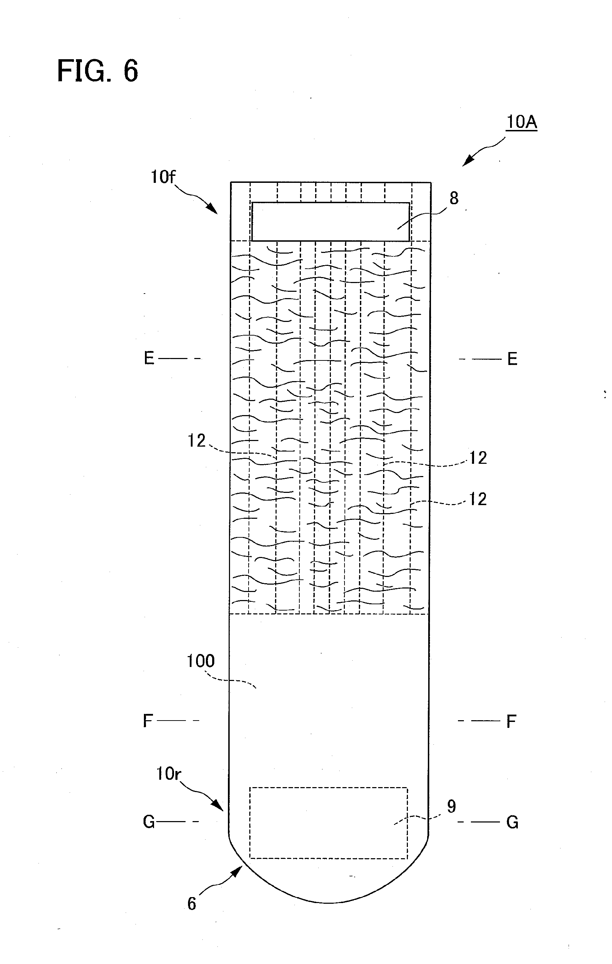

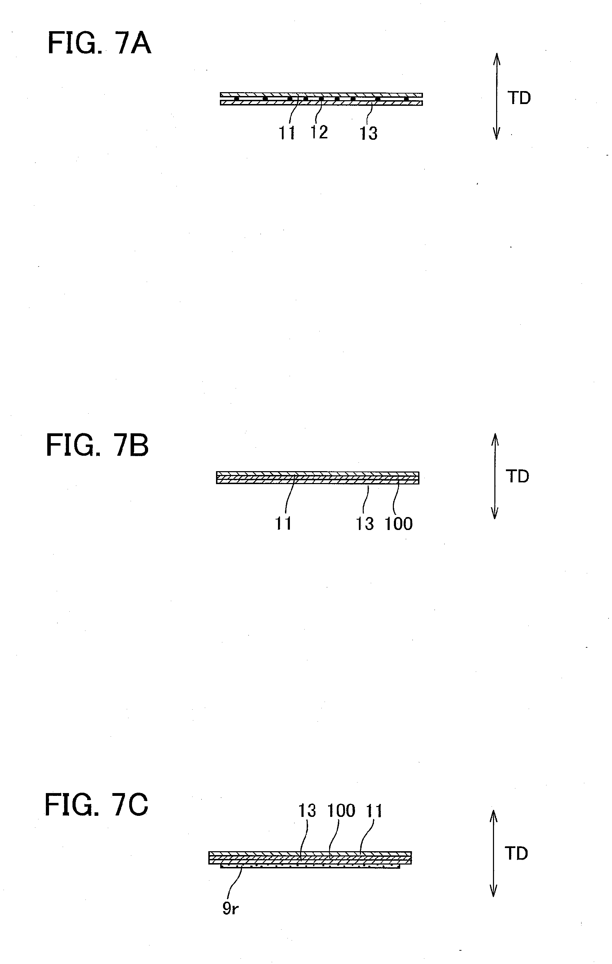

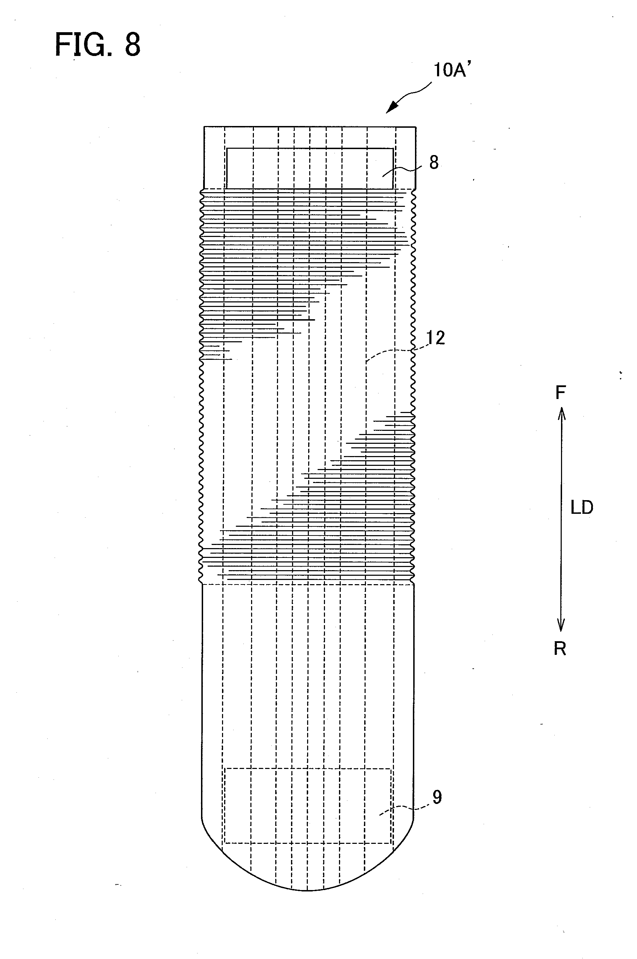

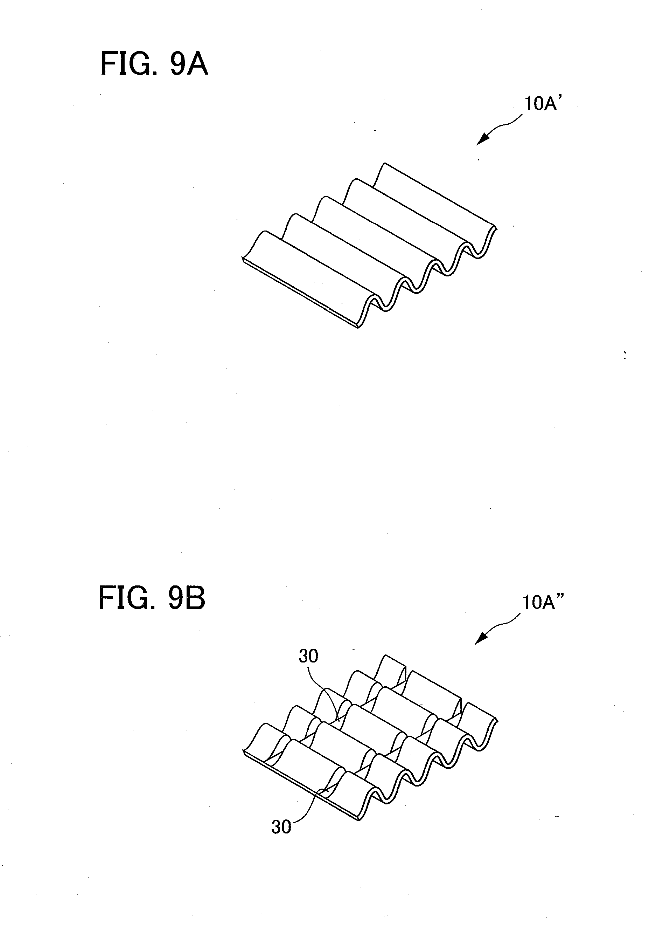

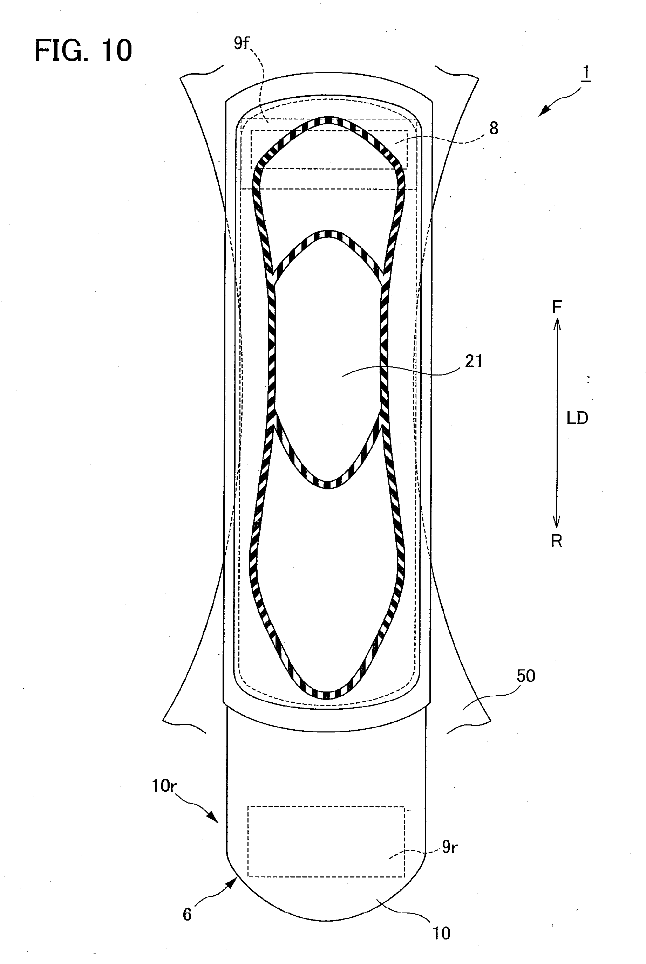

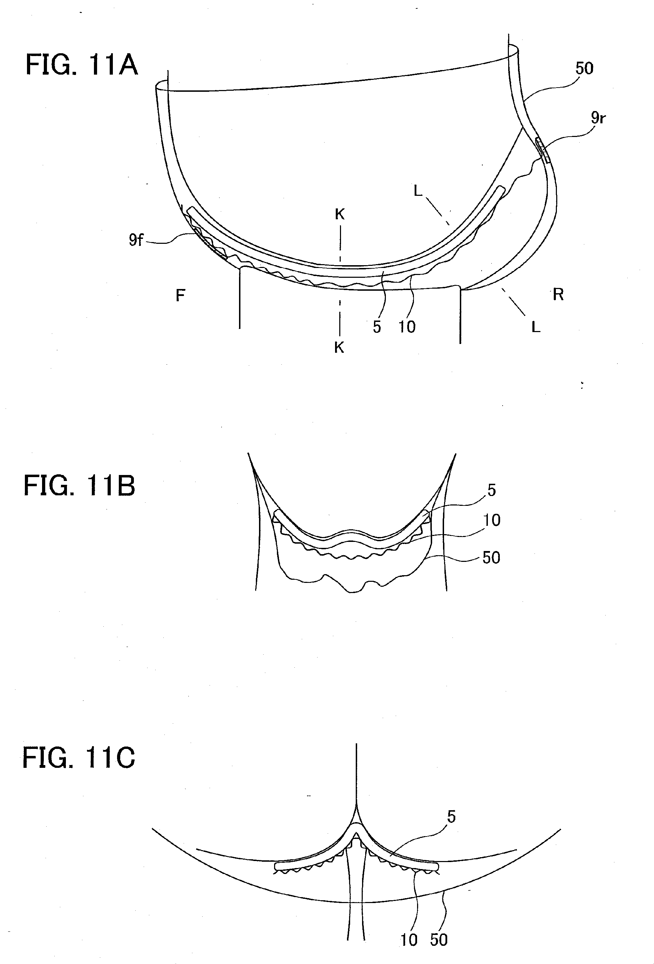

Fig. 1 is a front view of a sanitary napkin according to a first preferred embodiment of the present invention. Fig. 2 is a back view of Fig. 1. Fig. 3 is a sectional view taken along the line A-A of Fig. 1. Fig. 4A is a sectional view taken along the line B-B of Fig. 1. Fig. 4B is a sectional view taken along the line C-C of Fig. 1. Fig. 4C is a sectional view taken along the line D-D of Fig. 1. Fig. 5 is a front view of a belt-shaped member according to the first preferred embodiment. Fig. 6 is a front view showing another embodiment of the belt-shaped member. Fig. 7A is a sectional view taken along the line E-E of Fig. 6. Fig. 7B is a sectional view taken along the line F-F of Fig. 6. Fig. 7C is a sectional view taken along the line G-G of Fig. 6. Fig. 8 is a diagram showing still another embodiment of the belt-shaped member. Fig. 9A is a partially enlarged view of Fig. 8. Fig. 9B is a partially enlarged view showing yet another embodiment of the belt-shaped member. Fig. 10 is a front view showing the configuration when attaching the sanitary napkin according to the first preferred embodiment. Figs. 11A to 11C are diagrams showing the attached state of the sanitary napkin according to the first preferred embodiment.

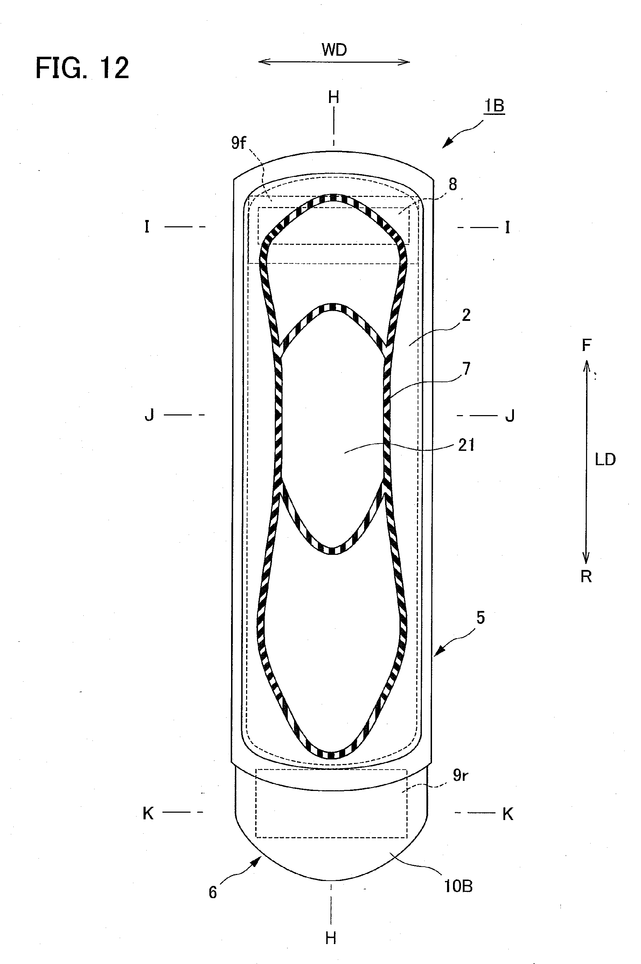

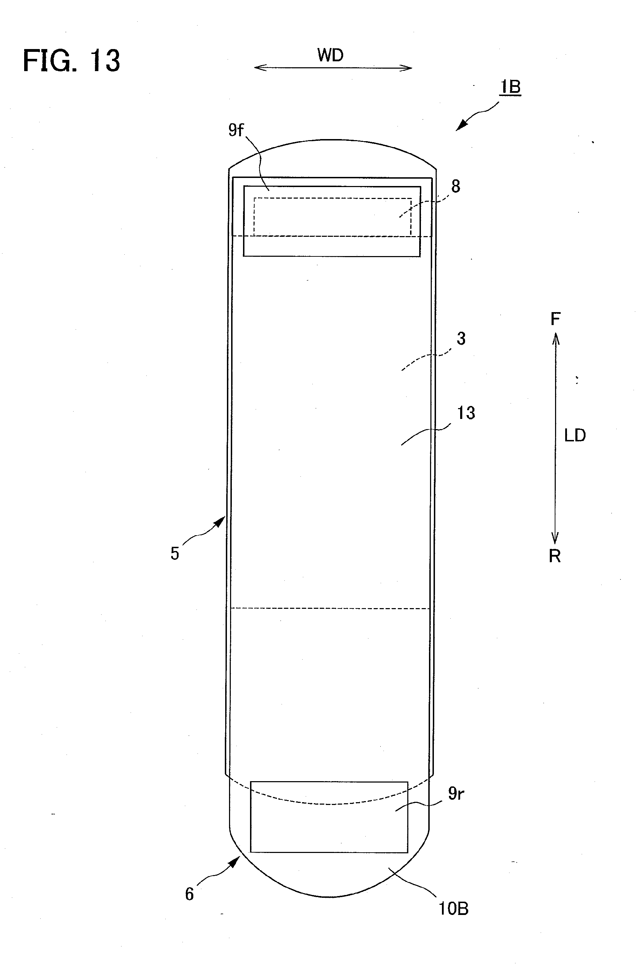

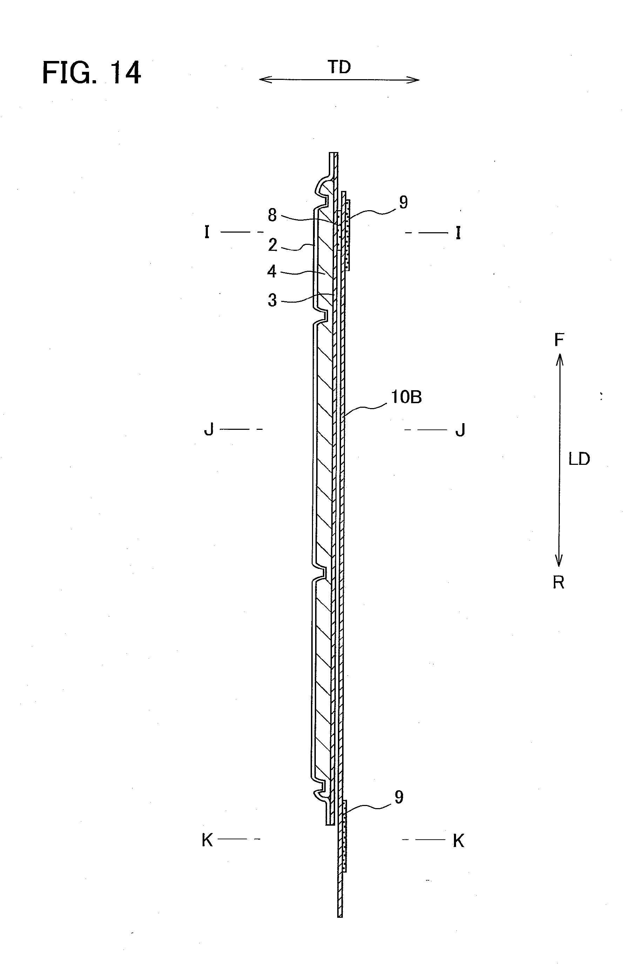

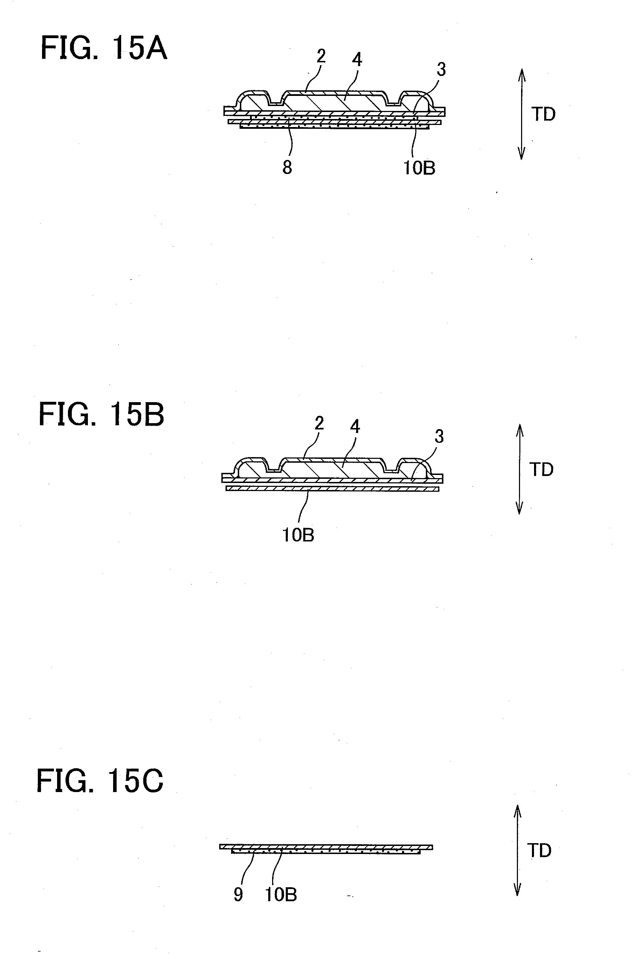

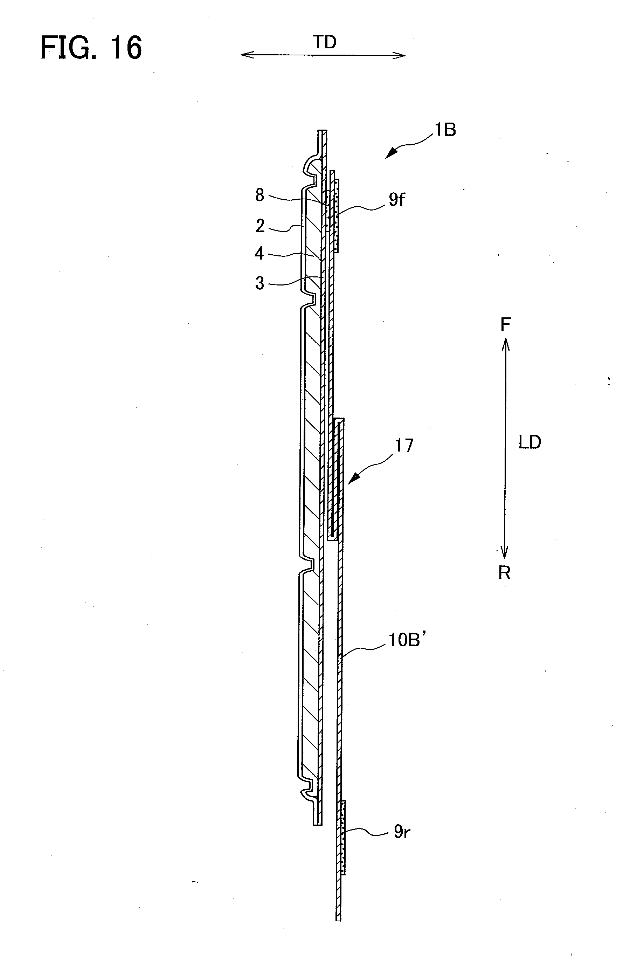

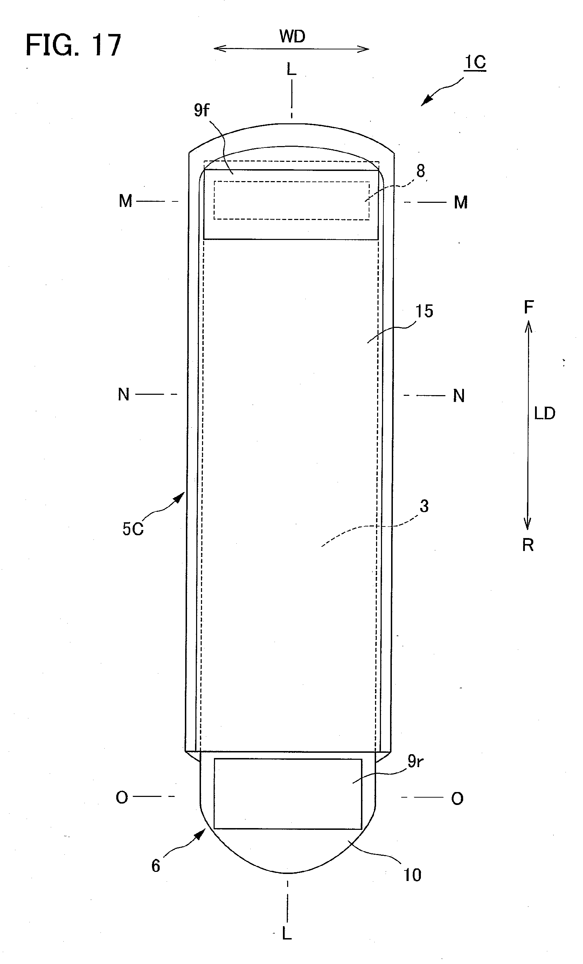

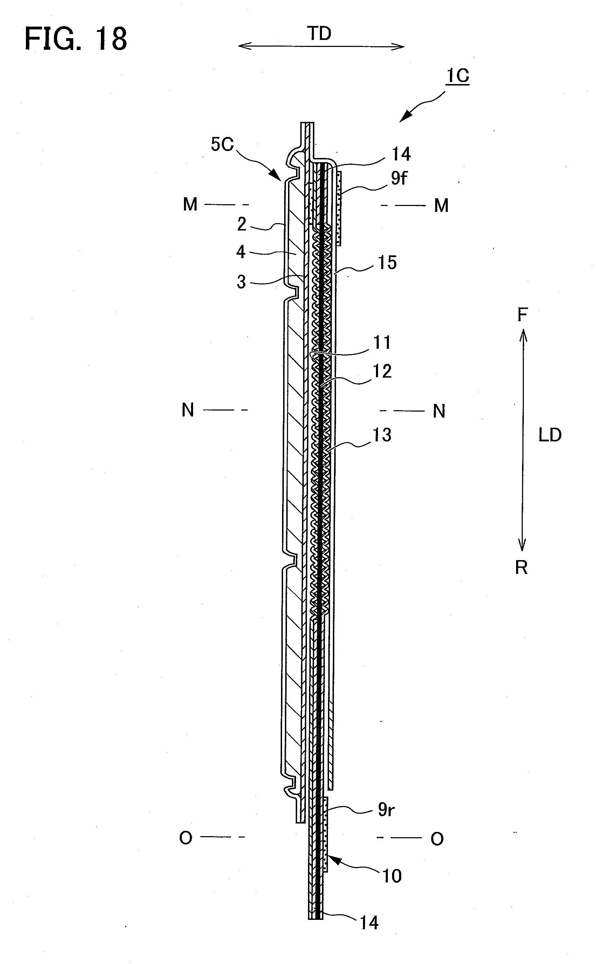

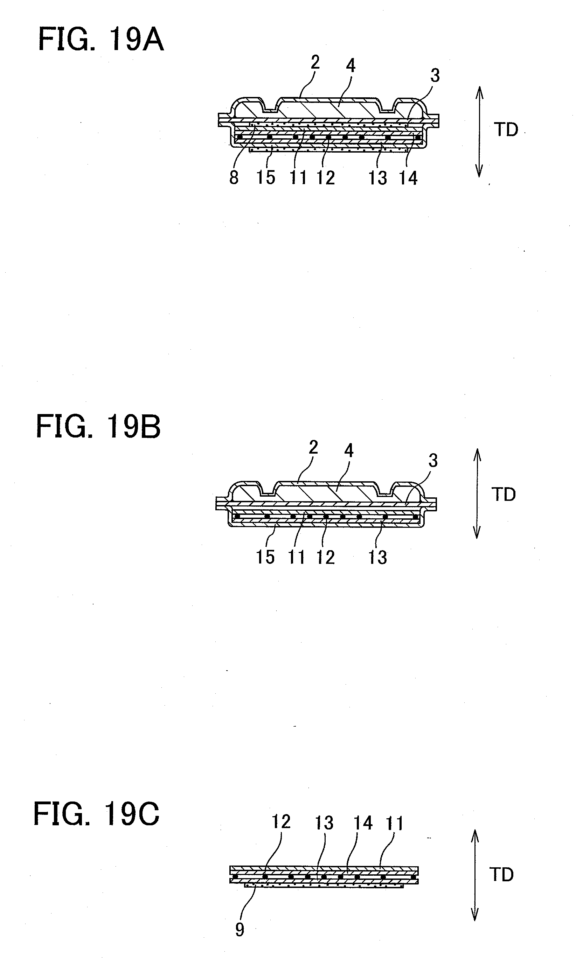

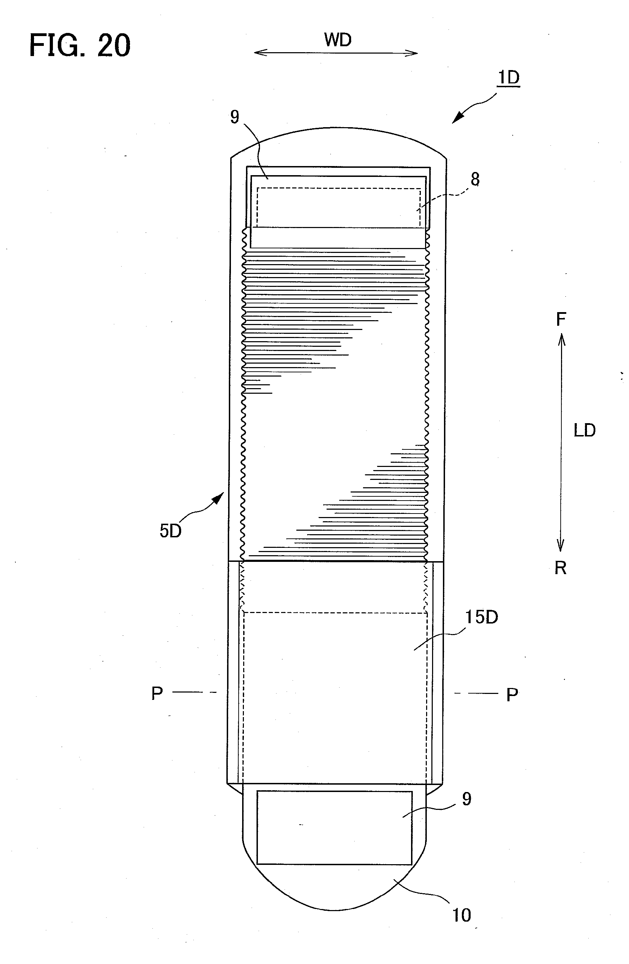

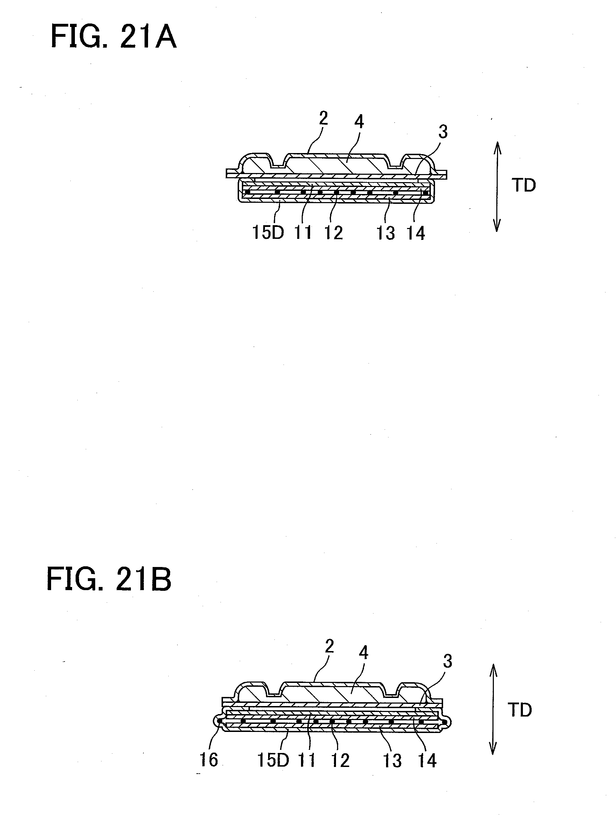

Fig. 12 is a front view of a sanitary napkin according to a second preferred embodiment of the present invention. Fig. 13 is a back view of Fig. 12. Fig. 14 is a sectional view taken along the line H-H of Fig. 12. Fig. 15A is a sectional view taken along the line I-I of Fig. 12. Fig. 15B is a sectional view taken along the line J-J of Fig. 11. Fig. 15C is a sectional view taken along the line K-K of Fig. 12. Fig. 16 is a sectional view taken along the line H-H, showing a modification of the sanitary napkin according to the second preferred embodiment. Fig. 17 is a front view of a sanitary napkin according to a third preferred embodiment of the present invention. Fig. 18 is a sectional view taken along the line L-L of Fig. 17. Fig. 19A is a sectional view taken along the line M-M of Fig. 17. Fig. 19B is a sectional view taken along the line N-N of Fig. 17. Fig. 19C is a sectional view taken along the line O-O of Fig. 17. Fig. 20 is a front view of a sanitary napkin according to a fourth preferred embodiment of the present invention. Fig. 21A is a sectional view taken along the line P-P according to another embodiment of Fig. 20. Fig. 21B is a sectional view taken along the line P-P of Fig. 20.

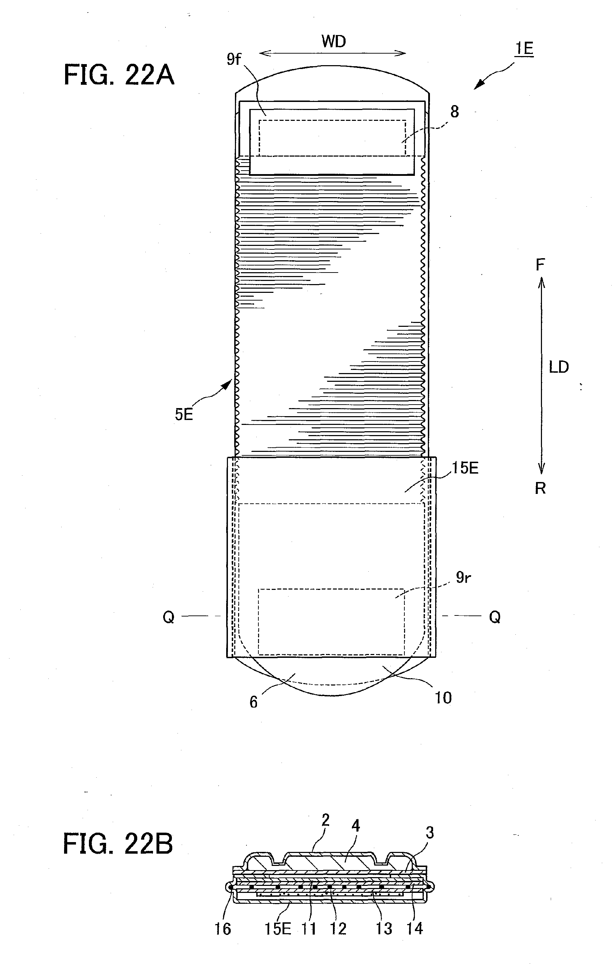

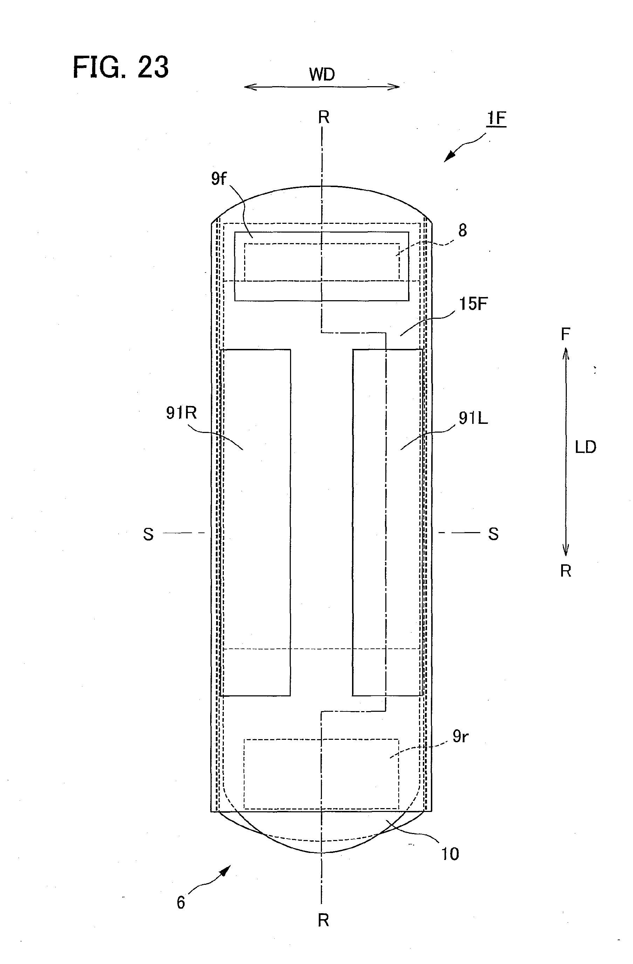

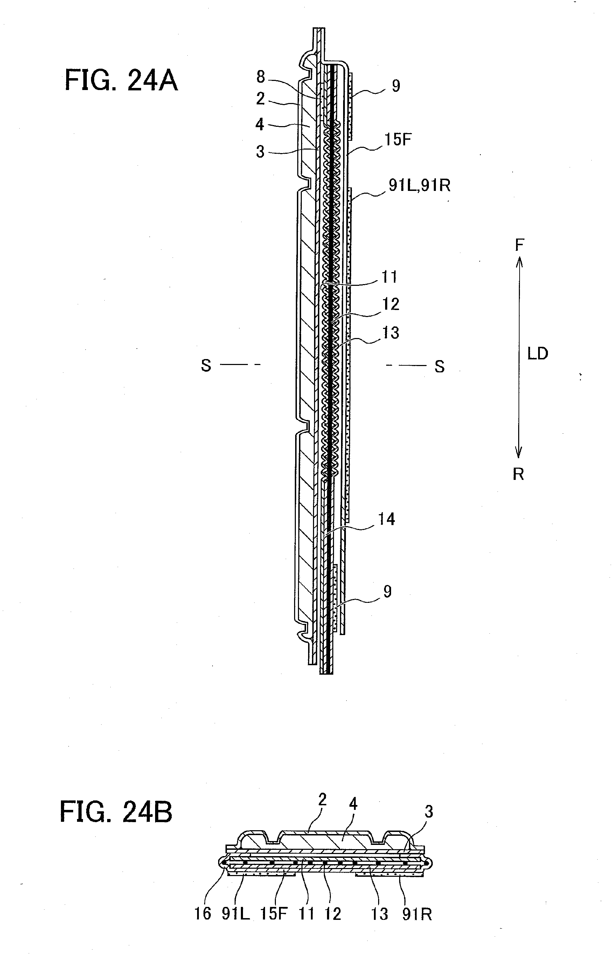

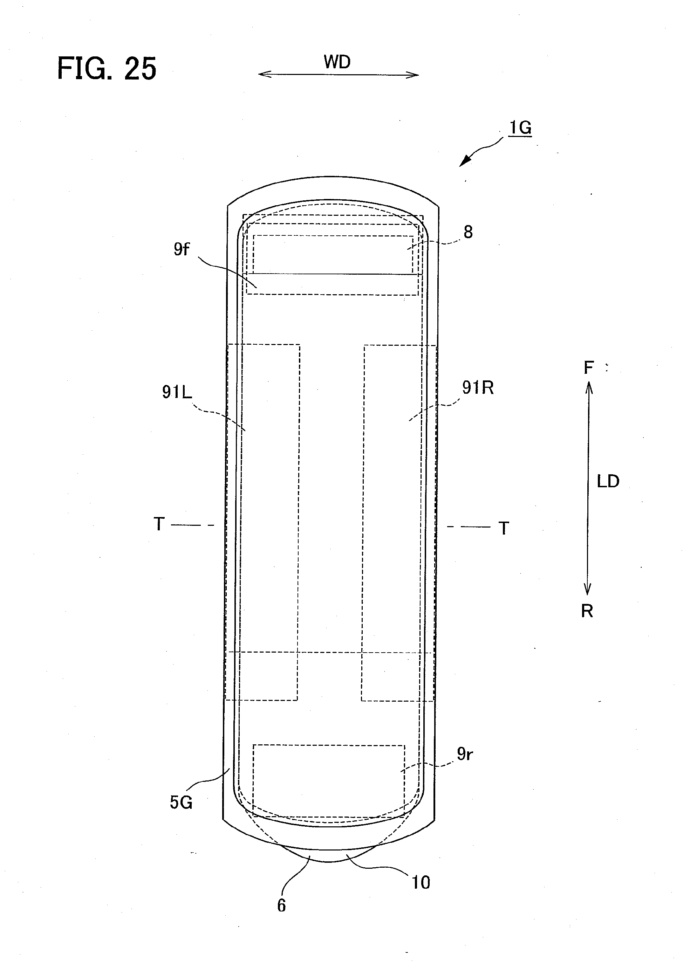

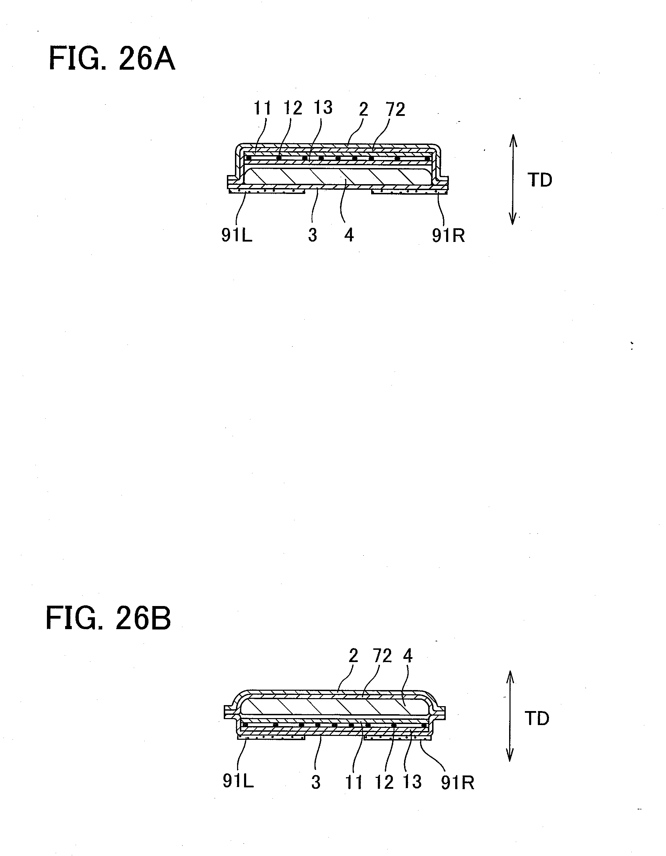

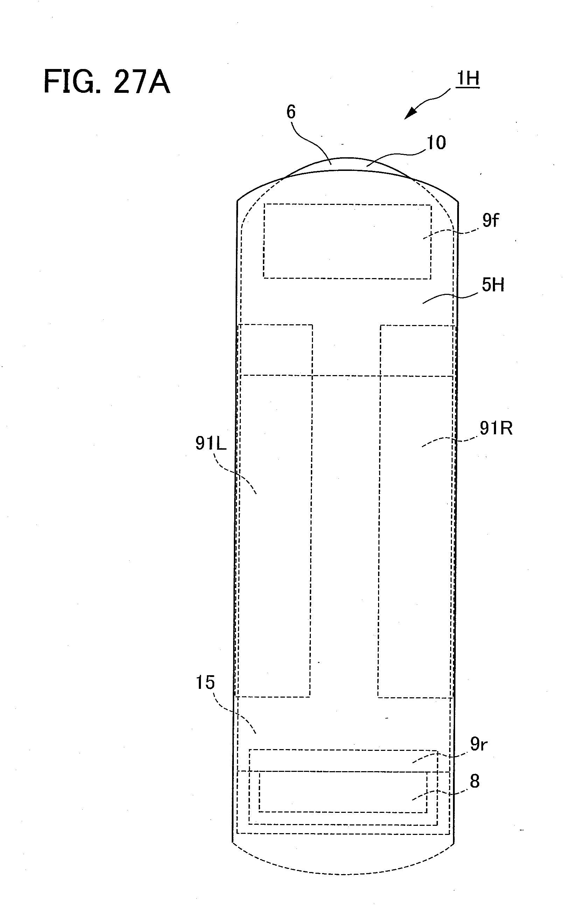

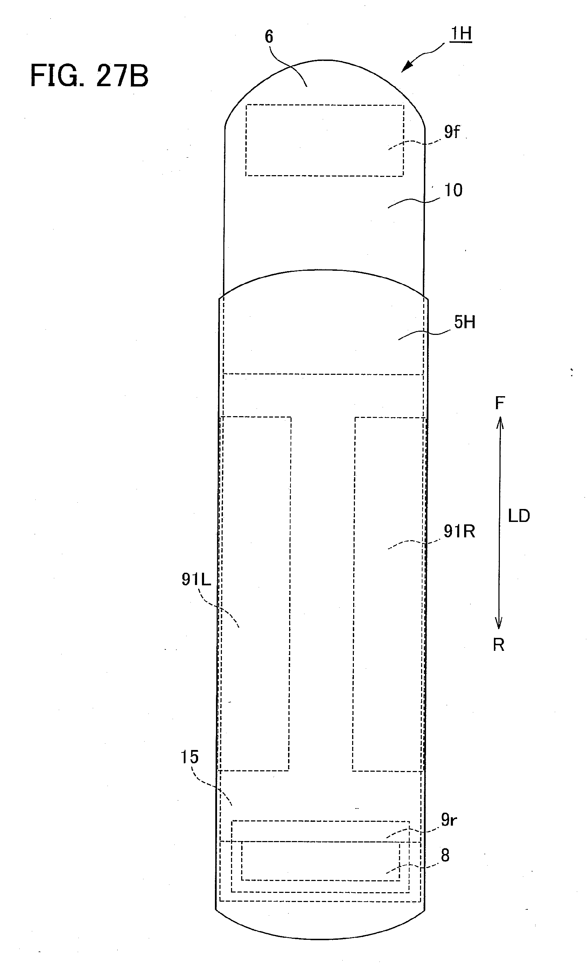

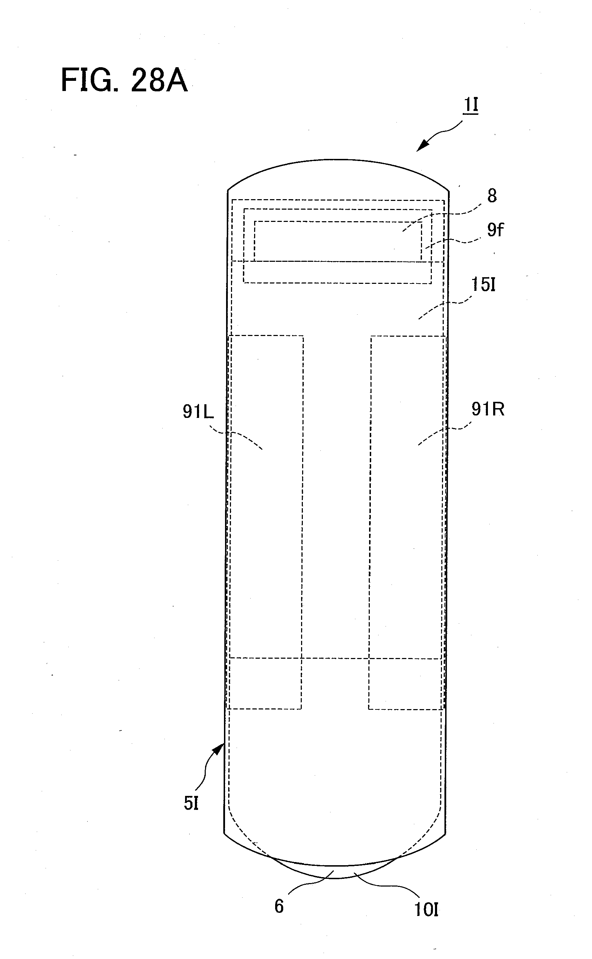

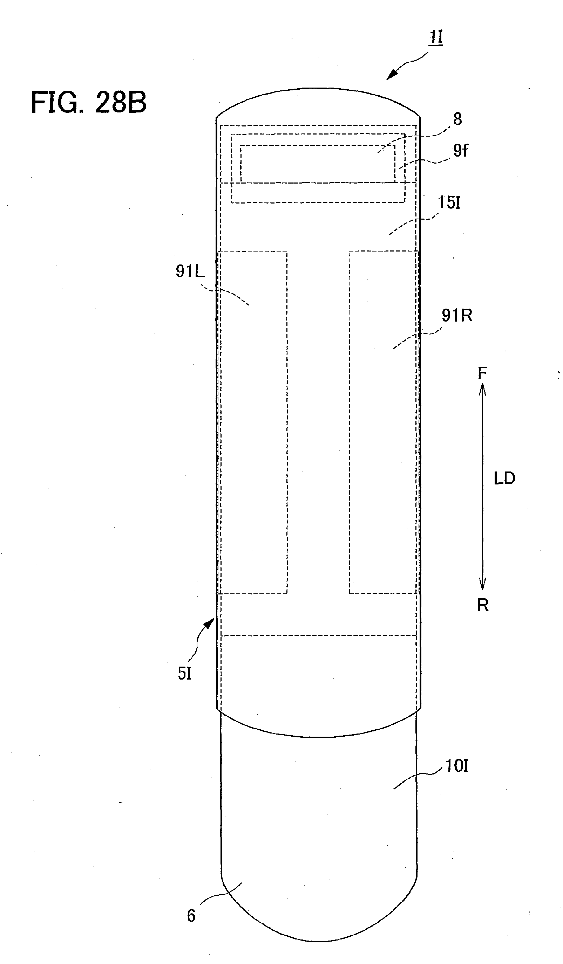

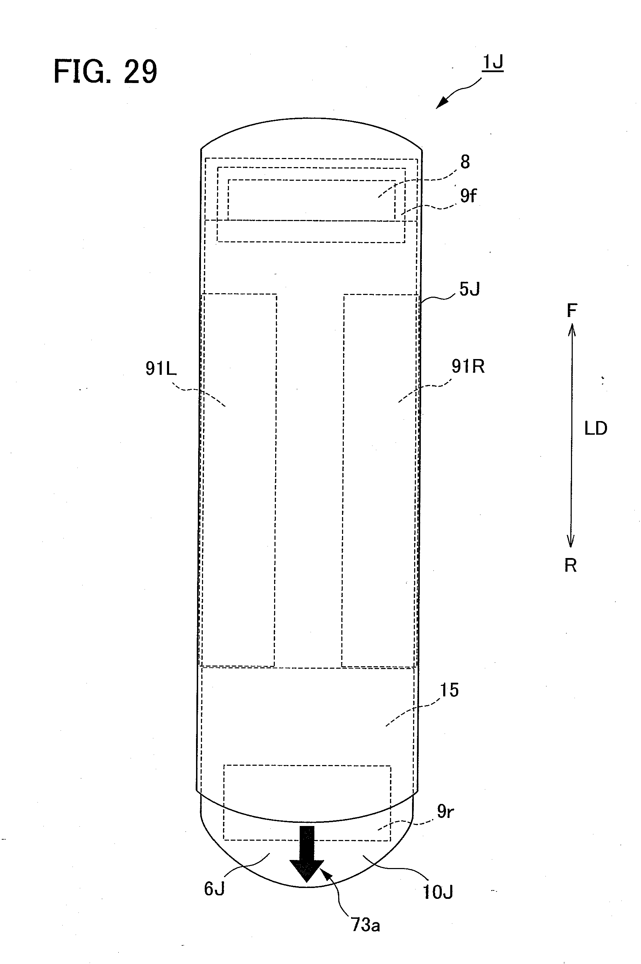

Fig. 22A is a front view of a sanitary napkin according to a fifth preferred embodiment of the present invention. Fig. 22B is a sectional view taken along the line Q-Q of Fig. 22A. Fig. 23 is a front view of a sanitary napkin according to a sixth preferred embodiment of the present invention. Fig. 24A is a sectional view taken along the line R-R of Fig. 23. Fig. 24B is a sectional view taken along the line S-S of Fig. 24A. Fig. 25 is a front view of a sanitary napkin according to a seventh preferred embodiment of the present invention. Fig. 26A is a sectional view taken along the line T-T of Fig. 25. Fig. 26B is a diagram showing a modification of the cross section taken along the line T-T of Fig. 25. Figs. 27A and 27B are front views of a sanitary napkin according to an eighth preferred embodiment of the present invention. Figs. 28A and 28B are front views of a sanitary napkin according to a ninth preferred embodiment of the present invention. Fig. 29 is a front view of a sanitary napkin according to a tenth preferred embodiment of the present invention.

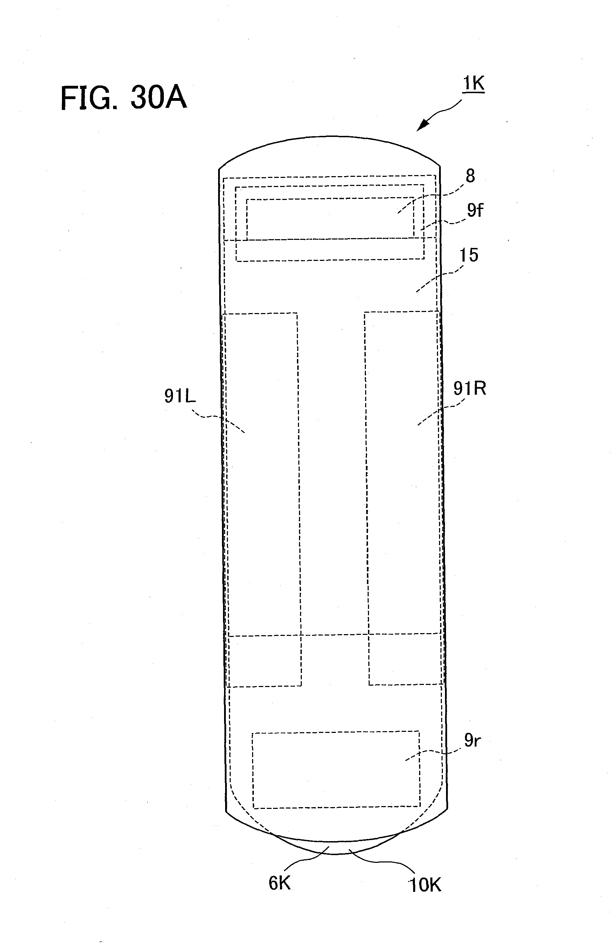

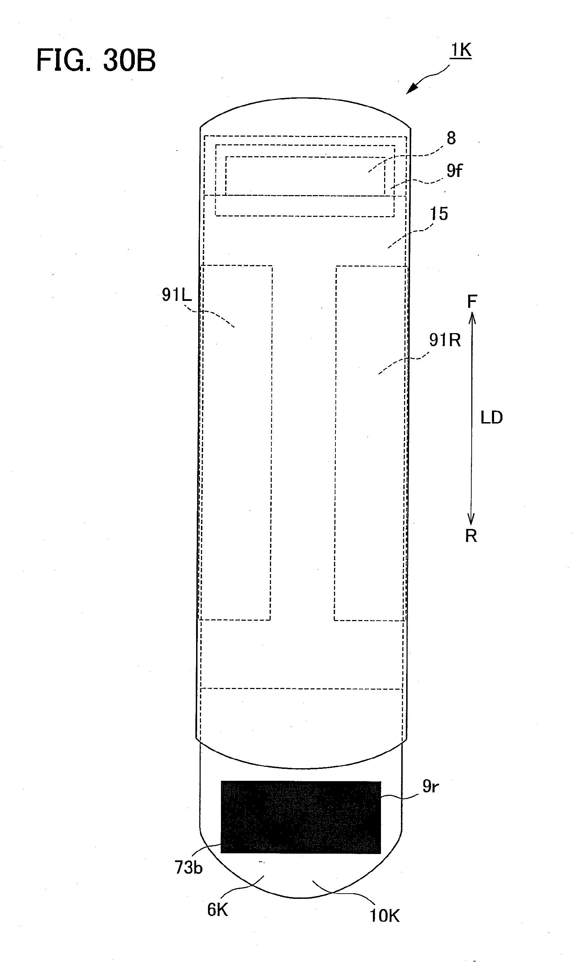

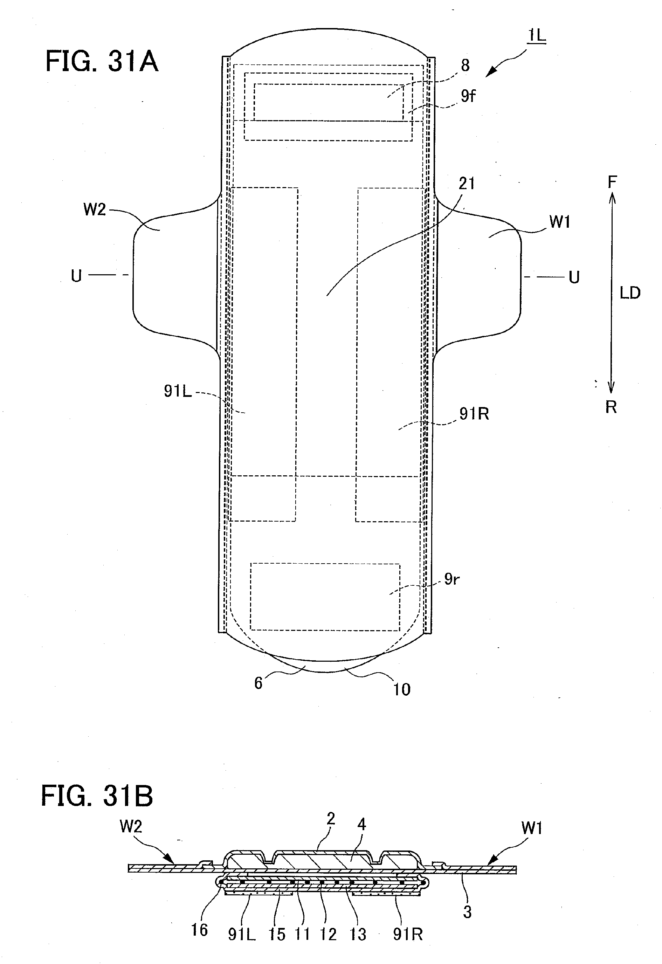

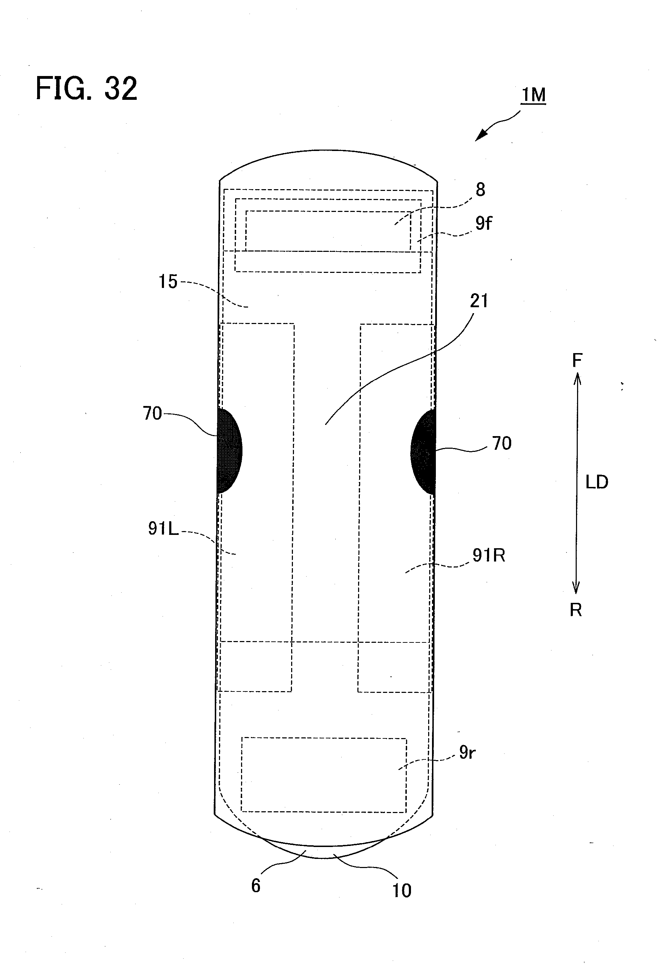

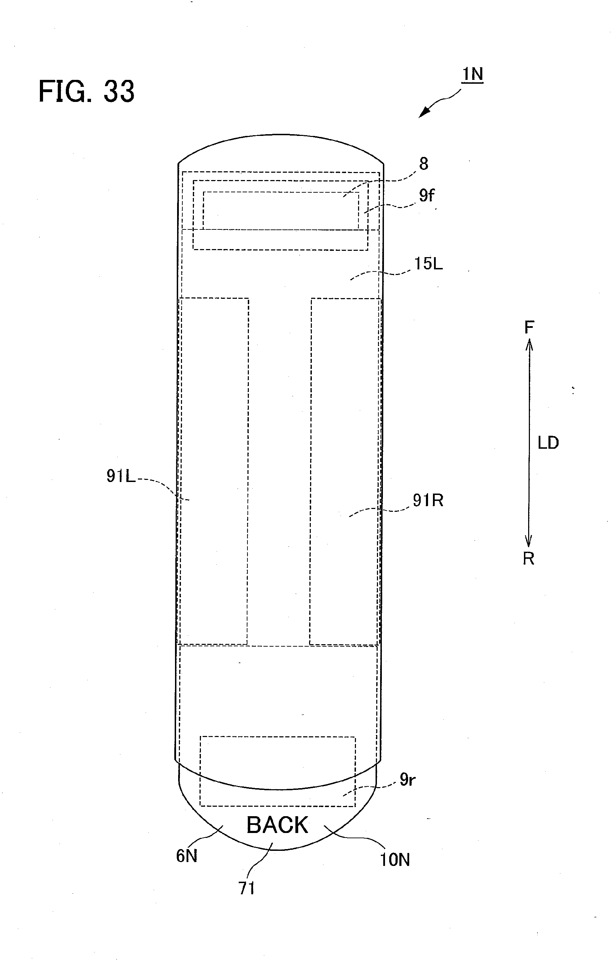

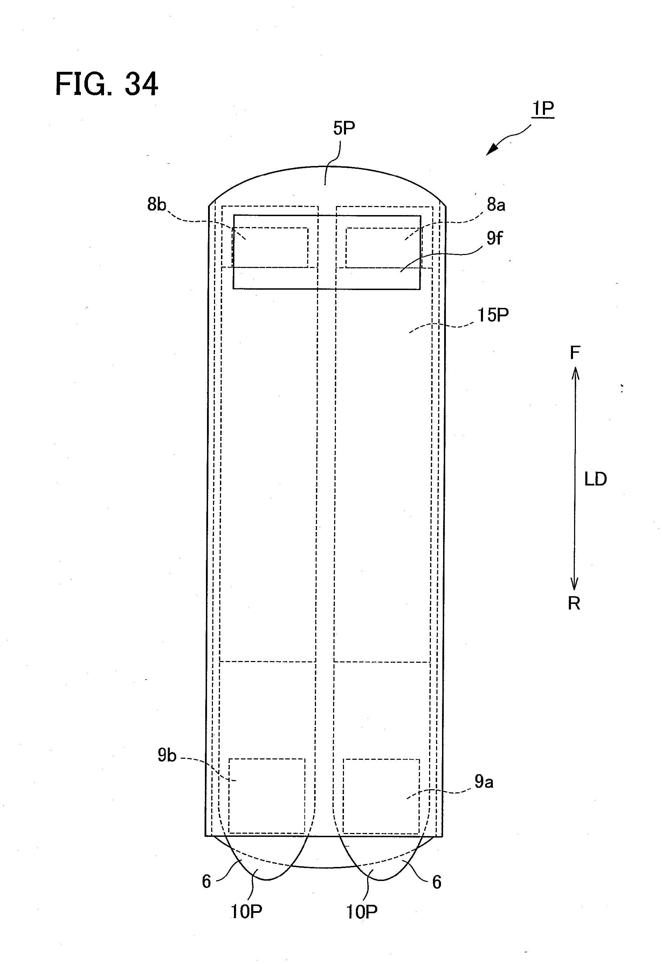

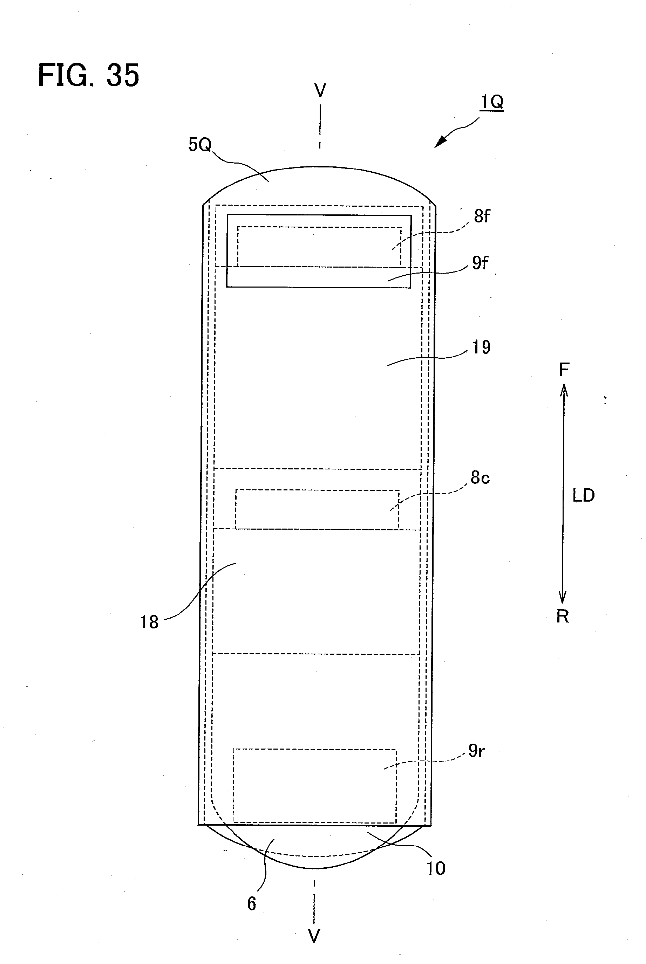

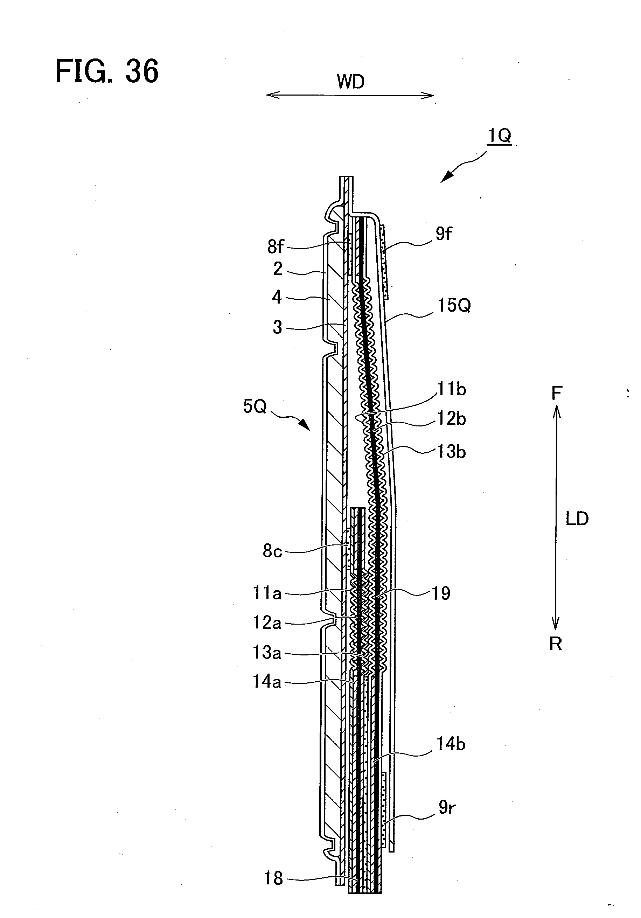

Figs. 30A and 30B are front views of a sanitary napkin according to an eleventh preferred embodiment of the present invention. Fig. 31A is a front view of a sanitary napkin according to a twelfth preferred embodiment of the present invention. Fig. 31B is a sectional view taken along the line U-U of Fig. 31A. Fig. 32 is a front view of a sanitary napkin according to a thirteenth preferred embodiment of the present invention. Fig. 33 is a front view of a sanitary napkin according to a fourteenth preferred embodiment of the present invention. Fig. 34 is a front view of a sanitary napkin according to a fifteenth preferred embodiment of the present invention. Fig. 35 is a front view of a sanitary napkin according to a sixteenth preferred embodiment of the present invention. Fig. 36 is a sectional view taken along the line V-V of Fig. 35.

### 1. First Embodiment

### 1-1. Overall Configuration

The overall configuration of the absorbent article of the present invention is described based on a sanitary napkin 1 according to a first preferred embodiment of the present invention. The sanitary napkin 1 as the absorbent article in the first preferred embodiment has a sanitary napkin body 5 that is an absorbent article body, and a belt-shaped member 10. Specifically, as shown in Fig. 1 to Fig. 4C, the sanitary napkin 1 has the sanitary napkin body 5 and the belt-shaped member 10. The sanitary napkin body 5 has a liquid permeable top sheet portion 2 which constitutes a surface layer and is disposed on the skin contact surface of a wearer, a liquid impermeable back sheet portion 3 which constitutes a back layer and is disposed on the non-skin contact surface of the wearer, and a liquid retainable absorbent body portion 4 which constitutes an absorption layer and is interposed between the top sheet portion 2 and the back sheet portion 3. The belt-shaped member 10 is disposed on the non-skin contact surface of the back sheet portion 3, and arranged along a longitudinal direction LD (Machine Direction) of the absorbent article body, on the other side of the absorbent article body in a thickness direction.

The sanitary napkin body 5 and the belt-shaped member 10 are connected to each other at a connecting portion 8 provided on one end in the longitudinal direction LD (MD). More specifically, one end of the belt-shaped member 10 is connected to the back sheet portion 3 at a predetermined position of a front region (F) of the sanitary napkin body 5, and the other end is provided with a free end 10r which extends by using the connecting portion 8 in a rear region (R) as the origin, along the longitudinal direction LD (MD) of the sanitary napkin body 5. As used here, the term "front region (f)" indicates the region extending from the vaginal opening to the abdominal part of the wearer when the sanitary napkin is worn. The term "rear region (R)" indicates the region extending from the vaginal opening to the buttocks of the wearer when the sanitary napkin is worn.

Although a connecting material is used to connect the sanitary napkin body 5 and the belt-shaped member 10, these may be connected to each other by applying pressure or the like.

The free end 10r of the belt-shaped member 10 extends out of the outer edge of the sanitary napkin 5 in the longitudinal direction LD (MD). The portion so extended is provided with a grip portion 6. Engaging portions 9f and 9r to be engaged with underwear 50 or the like are disposed on the non-skin contact surface of the free end 10r and on the non-skin contact surface of the belt-shaped member 10 in the connecting portion 8, respectively.

The sanitary napkin 1 of the present embodiment is further provided with a leak-proof groove portion 7 that can be formed by continuously compressing the top sheet portion 2 and the absorbent body portion 4.

### 1-2. Sanitary Napkin Body

The sanitary napkin body 5 is formed in a substantially elongated-shape. Shape of the sanitary napkin body 5 are, for example, a rectangle, ellipse, and guitar-shape, as well as one equipped with so-called wings W1 and W2 as described later, which prevent dislocation from the underwear 50. The present invention may employ any shape suitable for the body of the wearer and the shape of the underwear 50. The length of the sanitary napkin body 5 in the longitudinal direction LD (MD) is, for example, from 100 mm to 500 mm, preferably from 150 mm to 350 mm. The length in the width direction WD (Cross Direction) is, for example, from 30 mm to 200 mm, preferably from 40 mm to 180 mm.

The sanitary napkin body 5 has a liquid permeable region 21 formed in a substantially elliptical-shape at the substantially central portion in the width direction WD (CD) of the sanitary napkin body 5. The liquid permeable region 21 is surrounded by the leak-proof groove portion 7. The leak-proof groove portion 7 partitions the liquid permeable region 21. Excrement discharged from the excretory part, such as menstrual blood, passes through the top sheet portion 2 in the liquid permeable region 21, and is then absorbed by the absorbent body portion 4. Since the back sheet portion 3 disposed on the non-skin contact surface is liquid impermeable, the excrement can be absorbed by the absorbent body portion 4 and retained as is, without reaching the non-skin contact surface. A compressed portion can be formed in the leak-proof groove portion 7 by exposure to high compression and low compression continuously or with predetermined spaced intervals, so as to surround the liquid permeable region 21. The leak-proof portion 7 prevents mainly the leakage of the excrement such as menstrual blood absorbed by the absorbent body portion 4, and prevents the spread of the excrement in the absorbent body portion 4.

### 1-3. Belt-Shaped Member

As shown in Fig. 5, the belt-shaped member 10 is formed in a substantially elongated-shape. The belt-shaped member 10 can be attained by sandwiching a plurality of thread-shaped elastic members 12 between a pair of belt-shaped base material sheets 11 and 13, and by bonding the base material sheets 11 and 13, respectively. More specifically, the belt-shaped member 10 can be formed in the following steps of extending the elastic member 12 to a predetermined length by applying a predetermined tension in the longitudinal direction LD (MD) of the elastic member 12, and sandwiching the extended elastic member 12 with the base material sheets 11 and 13, followed by bonding.

The length of the belt-shaped member 10 in the width direction WD (CD) is preferably, for example, in the range of 30% to 150%, more preferably in the range of 60% to 130% of the length of the sanitary napkin body 5 in the width direction WD (CD). This is because when it is below 30% of the length of the sanitary napkin body 5 in the width direction WD (CD), for example, the sanitary napkin body 5 cannot be sufficiently adhered to the human body even if the belt-shaped member 10 is extended. This is also because when it is above 150%, the area to contact the femoral region of the wearer is excessive, and skin irritation or the like might occur by frictional contact with the femoral region.

The length of the belt-shaped member 10 in the longitudinal direction LD (MD) is preferably, for example, in the range of 30% to 300%, and more preferably in the range of 70% to 150% of the length of the sanitary napkin body 5 in the longitudinal direction LD (MD). This is because when it is below 30% of the length of the sanitary napkin body 5 in the longitudinal direction LD (MD), the sanitary napkin body 5 cannot sufficiently contact with the human body, even if the belt-shaped member 10 is extended. This is also because when it is above 150%, the area to contact with the femoral region of the wearer is excessive, and skin irritation or the like might occur by frictional contact with the femoral region. Moreover, there is the likelihood that the belt-shaped member 10 in the extended state cannot be attached to the underwear 50, and it cannot make contact with the excretory part.

The extensible range of the belt-shaped member 10 is preferably, for example, in the range of 105% to 300%, more preferably in the range of 110% to 180%, when the non-extended state of the elastic member 12 is 100%. This is because when the extended state is less than 105%, the force with which the sanitary napkin body 5 can be pressed against the human body is weak, and it is difficult to establish the structure for lifting the sanitary napkin body 5. This is also because when it is larger than 300%, more pressing force to the human body than necessary is developed, and the wearer might feel uncomfortable. The force of the elastic material 12 when the extended state is 105% to 300% in extension rate is preferably, for example, in the range of from 5 cN / 25 mm to 500 cN / 25 mm, and more preferably from 20 cN / 25 mm to 100 cN / 25 mm.

The belt-shaped member 10 has the grip portion 6 at the free end 10r on the other end opposite one end connected to the sanitary napkin body 5, in the longitudinal direction LD (MD). The grip portion 6 is disposed so as to extend past the outer edge of the sanitary napkin body 5. In order to facilitate the grip of the grip portion 6 by the wearer, a sheet member 14 having a predetermined strength may be interposed between the base material sheets 11 and 13, at a position corresponding to the grip portion 6 of the belt-shaped member 10. The grip portion 6 may be extended to a degree that the wearer can grip it with their fingers or the like. Preferably, the grip portion 6 is disposed at an inextensible region.

Thus, the belt-shaped member 10 provided with the grip portion 6 enables the wearer to easily recognize the belt-shaped member 10, enabling the prevention of misattachment at the time of the attachment.

As shown in Figs. 6 to 7C, in a belt-shaped member 10A, an absorptive member 100 may be interposed between the base material sheets 11 and 13, at a free end 10r including the grip portion 6. Preferably, the absorptive member 100 is also arranged at a region to which the belt-shaped member 10A extends from the outer edge of the sanitary napkin body 5 when the belt-shaped member 10A is extended. With this arrangement, even if the excrement such as menstrual blood leaks from the sanitary napkin body 5 and effuses along the wearer's buttocks when attaching the sanitary napkin 1, it can be absorbed by the absorptive member 100 extending from the sanitary napkin body 5. Additionally, the absorptive member 100 so arranged enables the belt-shaped member 10A to also absorb the excrement that directly effuses along the wearer's buttocks.

In the region where the absorptive member 100 is provided, it is preferable to provide a liquid impermeable sheet (not shown) on the non-skin contact surface of the belt-shaped member 10A, in the region corresponding to the absorptive member 100. This liquid impermeable sheet is preferably disposed so as to cover the outer edge on the free end 10r of the belt-shaped member 10A. Furthermore, the liquid impermeable sheet is disposed so as to cover at least a portion on each of both sides of the belt-shaped member 10A. This can prevent the excrement absorbed by the absorptive member 100 from soaking through the underwear 50 to be engaged.

When in the belt-shaped number 10, the extended elastic member 12 is bonded with it interposed between the base material sheets 11 and 13, and then the extension of the elastic member 12 is released, an extension allowance can be formed in the base material sheets 11 and 13. The extension allowance enables the belt-shaped member 10 to be extended. The term "extension allowance" includes sag and looseness developed in the base material sheets 11 and 13 when these are shrunk by releasing the extension of the elastic member 12 connected to the base material sheets 11 and 13. The belt-shaped member 10 can be extended by the amount of the sag or the like. In addition, the belt-shaped member 10, in which the elastic member 12 is interposed between the inflexible base material sheets 11 and 13, is able to prevent so-called neck in, namely the phenomenon that, for example, when a belt-shaped elastic member is extended, the substantially central portion thereof becomes narrow.

As other extension process of the belt-shaped member 10, it may be made extensible by performing a partial slitting process or embossing finish.

Fig. 8 and Fig. 9A show a belt-shaped member 10A' subjected to corrugated embossing finish. As shown in Fig. 8 and Fig. 9A, the corrugated embossing finish produces a corrugated pattern where concavities and convexities are continuously formed in the longitudinal direction LD (MD). The pitch between the convexities is, for example, from 0.5 mm to 5.0 mm. The height of the convex portion is, for example, from 0.5 mm to 5.0 mm. The clearance between the convex side surfaces in meshing engagement is, for example, from 0 mm to 3.0 mm.

The corrugated embossing can be formed by interposing the base material sheets 11 and 13 between a pair of emboss rolls provided with a lower roll portion having on the surface thereof a continuous concave portion, and an upper roll portion having on the surface thereof a continuous convex portion, and allowing the concave portion and the convex portion to mesh with the base material sheets 11 and 13. This enables extensibility to be imparted. When employing the corrugated embossed finish, the belt-shaped member 10A' may be formed by interposing the elastic member 12 between the base material sheets 11 and 13, in the state where the elastic member 12 is extended or not extended.

The belt-shaped member 10 thus formed can facilitate setting of the extension range thereof. For example, when the extension range is set to 130% of the non-extended state, a base material sheet may be used which has a length of 130% when it is extended, and the concave and convex shapes of corrugated embossing may be formed in this base material sheet. Alternatively, the elastic material 12 having a maximum extension of 130% may be interposed in the extended state, in between the base material sheets 11 and 13. For example, when the extension range is 130% of the non-extended state, the wearer can easily pull the belt-shaped member 10 up to 130%, and excess force is required to pull further. Thus, the belt-shaped member 10 enables the wearer to recognize the extension range by providing a increased-pull-resistance point in the extension range for the wearer to feel. By setting the extension range to the belt-shaped member 10, the sanitary napkin 1 can be put on properly by the user.

In order to impart regularity to the extension allowance, blank 30 (areas having neither a concave nor a convex) may be applied partially in the width direction WD (CD), to the corrugated embossing pattern of the base material sheets 11 and 13 in a belt-shaped member 10A". Specifically, in addition to the embossing pattern shown in Fig. 5, blanks 30 having a width of 0.5 mm to 3.0 mm may be formed at pitches of 5 mm to 40 mm in the width direction WD (CD) (refer to Fig. 9B). Alternatively, the blanks 30 may be formed by coating a base material with a hot melt in the coating pattern that coating is carried out with continuously in the width direction WD (CD), and repeating the coating and non-coating in the longitudinal direction LD (MD).

Although the present embodiment employs the inextensible material as the base material sheets 11 and 13, without limiting to this, the present invention may employ, for example, an extensible material or a flexible material. Although in the present embodiment, the belt-shaped member 10 is made up of the base material sheets 11 and 13 and the elastic material 12, without limiting to this, the present invention may employ only a flexible base material sheet. For example, it is possible to employ a fibrous sheet using a thermoplastic elastomer resin, a nonwoven fabric obtained by mixing urethane fiber and a synthetic fiber, anonwoven fabric including a layer obtained by forming elastomer resin by melt blown, film sheet, or the like.

There is no need to provide the elastic material 12 in the entire region of the base material sheets 11 and 13, and it may be provided at least in a portion of the region. For example, the region of the engaging material as the engaging portion with the underwear 50 may be made up of the base material sheets 11 and 13, and the rest may be arranged so that the elastic member 12 is interposed between the sheets 11 and 13. Also, the region constituting the grip portion 6, as described later, may be made up of the base material sheets 11 and 13, without interposing the elastic material 12 therebetween.

The belt-shaped member 10 is also required to have a flexible region in at least a portion of the longitudinal direction LD (MD). Preferably, the belt-shaped member 10 is flexible at the position where the sanitary napkin body 5 is contacting the excretory part. More preferably, the belt-shaped member 10 is flexible at the position where the sanitary napkin body 5 is contacting the vaginal opening. That is, the belt-shaped member 10 may include an inflexible region in a portion of the longitudinal direction (LD), at the time of the attachment. For example, the region where the back sheet portion 3 and the belt-shaped member 10 are connected to each other, and the region may be inflexible where the sanitary napkin body 5 or the belt-shaped member 10 is engaged with the underwear 50, and the rest may be flexible. In the absence of any flexibility in the connecting portion 8 and the engaging portions 9f and 9r, the connecting material for connecting the back sheet portion 3 and the belt-shaped member 10, and the engaging material for engaging the belt-shaped member 10 and the underwear 50 are hard to come off.

The inflexible region may be formed, for example, by connecting the elastic material 12 in the non-extended state, with the base material sheets 11 and 13. The elastic material 12 in the extended state may be cut to eliminate elastic force. Alternatively, extensible regions generated by the corrugated embossing finish may be connected together to form the inflexible region. The base material sheets 11 and 13 in may inherently be inflexible without applying any process for forming the flexible region, such as the corrugated embossing finish of the base material sheets 11 and 13.

### 1-4. Position to Connect Sanitary Napkin Body and Belt-Shaped Member

The sanitary napkin body 5 and the belt-shaped member 10 are connected at the connecting portion 8. The position to connect the belt-shaped member 10 and the sanitary napkin body 5 is preferably in the front region (F) away from the wearer's vaginal opening during the time the sanitary napkin 1 is worn. For example, the position of connection is preferably located at a swelling portion present in the vicinity of the wearer's excretory part. More specifically, the position of connection is preferably a position corresponding to the pubis present in the vicinity of the excretory part. Since the position corresponding to the pubis is firmer than the surroundings and slightly swelled, the pressure from the underwear 50 can be increased thereby to restrain the sanitary napkin body 5 from slipping from the human body. Thus, even if the belt-shaped member 10 is pulled and extended at the time of the attachment, the sanitary napkin body 5 cannot be bent due to the force induced at that time. As a result, it is difficult for the sanitary napkin body 5 to slip at the time of the attachment and while the sanitary napkin 1 is worn.

In the connecting portion 8, the sheet member 14 having a predetermined strength may be interposed between the base material sheets 11 and 13. This can impart the predetermined strength to the connecting portion 8, enabling the connection of the belt-shaped member 10 to be stabilized.

### 1-5. Position to Engage Belt-Shaped Member and Underwear

The belt-shaped member 10 has the engaging portions 9f and 9r to be engaged with the underwear 50 on both ends in the longitudinal direction LD (MD), respectively. The engaging portion 9f is engaged with the underwear 50 or the like in the front region (F), and the engaging portion 9r is engaged with the underwear 50 in the rear region (R). Preferably, the engaging portion 9f is provided at a position opposing the connecting portion 8 where the sanitary napkin body 5 and the belt-shaped member 10 are connected to each other. This is because the connecting portion 8 for connecting the sanitary napkin body 5 and the belt-shaped member 10 is the origin of the belt-shaped member 10 to the free end 10r, and also the point where the tension of the belt-shaped member 10 to the sanitary napkin body 5 is maximized, whereby it is difficult for the sanitary napkin body 5 to slip from the underwear 50 at this point.

Preferably, the engaging portion 9r is located slightly ahead of the rearmost end in the rear end portion of the belt-shaped member 10. The reasons for this are as follows. Since the human body is often significantly curved from the vaginal opening to the rear, the belt-shaped member 10 lying in the rear side allows for the expansion of the flexible range of the belt-shaped member 10. The extension of the belt-shaped member 10 along the curvature of the human body facilitates the transmission of the force on the belt-shaped member 10 to the sanitary napkin body 5 located in the vicinity of the excretory part. In addition, by disposing the engaging portion 9r slightly ahead of the rearmost end in the rear end portion of the belt-shaped member 10, it is also possible to prevent the engaging material from being adhered to the finger or the like when the wearer grips the grip portion 6.

Examples of the engaging material used for the engaging portions 9f and 9r are a hot melt adhesive, hook material, and binder. The engaging material used for the engaging portion 9r disposed in the rear region (R) may be the engaging material to be engaged with the underwear 50 or the wearer's body. That is, the engaging portion may be engaged with the underwear 50 or the like, or alternatively engaged with the wearer's body.

In order to avoid the belt-shaped member 10 from slipping from the underwear or the like during the time the sanitary napkin 1 is worn, when the belt-shaped member 10 has flexibility, the engaging force of the engaging material is required to be larger than the contractive force of the belt-shaped member 10. Accordingly, the shear force that is the engaging force is preferably set to be larger than the contractive force in the range of 5 cN / 25 mm to 500 cN / 25 mm.

### 1-6. Manner of Application

Manners of application of the sanitary napkin 1 in the first preferred embodiment of the present invention are described with reference to Figs. 10 to 11C. More specifically, there are first and second manners of application of the sanitary napkin 1.

In the first manner of application, the sanitary napkin 1 is first arranged at a predetermined position of the underwear 50, and the engaging material disposed at the engaging portion 9f in the front region (F) on the non-skin contact surface of the sanitary napkin 1 is engaged with the underwear 50. More specifically, the engaging material is adhered to the underwear 50 (refer to Fig. 10). At this time, by removing a peelable sheet (not shown) on the non-skin contact surface of the engaging portion 9f, the engaging material can be exposed. The term "predetermined position of the underwear 50" includes, for example, an expected position where the liquid permeable region 21 in the sanitary napkin 1 can be brought into contact with the wearer's excretory part.

Subsequently, the grip portion 6 disposed on the free end 10r of the belt-shaped member 10 is pulled to the rear region (R) in the longitudinal direction LD (MD), while pressing the underwear 50 and the sanitary napkin 1 at the engaging portion 9f in the front region (F) of the sanitary napkin 1. As a result, the belt-shaped member 10 can be extended. If the sanitary napkin 1 is provided with the cover member 15 described later, the engaging portion 9r will appear at this time on the non-skin contact surface of the belt-shaped member 10. The engaging material applied to the engaging portion 9r is adhered to a predetermined position of the underwear 50 so that the extension portion of the belt-shaped member 10 corresponds to the wearer's excretory part. At this time, the belt-shaped member 10 is preferably adhered to the underwear 50 in the state where the belt-shaped member 10 is extended as much as possible.

On the completion of the attachment of the sanitary napkin 1, the underwear 50 is worn in this state so that the belt-shaped member 10 and the sanitary napkin 5 can be deformed in a gentle curve, and force can be transmitted from the belt-shaped member 10 to the sanitary napkin body 5 in the direction of the wearer's body. At this time, the belt-shaped member 10 upwardly presses the sanitary napkin body 5 so as to press it against the human body. As a result, the wearer's excretory part and the groove in the vicinity of the excretory part can contact the sanitary napkin 1 (refer to Figs. 11A to 11C).

In the second manner of application, the sanitary napkin 1 is first arranged at the same position as in the first manner of application, and fixed to the underwear 50. More specifically, the sanitary napkin 1 is arranged at a predetermined position of the underwear 50, and the engaging material disposed at the engaging portion 9f in the front region (F) on the non-skin contact surface of the sanitary napkin 1 is engaged with the underwear 50. In this state, the wearer is putting on the underwear 50. Subsequently, the wearer grips the grip portion 6 of the belt-shaped member 10 extending to the rear region (R) on the internal surface of the underwear 50, and engages the grip portion 6 with a predetermined position of the underwear 50 by pulling the grip portion 6 in the longitudinal direction LD (MD), along the curvature of the human body. If the sanitary napkin 1 is provided with the cover member 15 described later, by pulling the grip portion 6, the engaging portion 9r will appear disposed on the non-skin contact surface of the belt-shaped member 10, and the underwear 50 will be engaged therewith in the same manner. If the engaging portion 9r is provided with a removable sheet, the removable sheet may be removed in advance or removed after, or before pulling in the longitudinal direction LD (MD).

At this time, the underwear 50 is in the state of being lifted to the human body together with the sanitary napkin 1. Therefore, the free end 10r of the belt-shaped member 10 is pulled to engage the sanitary napkin 1 with the underwear 50, and the position can be adjusted so that the belt-shaped member 10 is adhered to the groove in the vicinity of the wearer's excretory part (refer to Figs. 11A to 11C).

Thus, since the underwear 50 and the belt-shaped member 10 are fixed to each other by gripping the grip portion 6 and pulling the belt-shaped member 10 to the rear region (R), tension can be exerted on the belt-shaped member 10 in the longitudinal direction LD (MD). When the belt-shaped member 10 is brought near the human body, the belt-shaped member 10 and the sanitary napkin 5 can be deformed in a gentle curve, and force can be transmitted from the belt-shaped member 10 to the sanitary napkin body 5 in the direction of the wearer's body. At this time, the belt-shaped member 10 pushes up the sanitary napkin body 5 so as to lift it to the human body. As a result, the wearer's excretory part and the groove in the vicinity of the excretory part can be close contact with the sanitary napkin 1.

In an alternative manner of application in the present embodiment, the belt-shaped member 10 may not be used. In this case, the belt-shaped member 10 can be used after the underwear 50 is put on.

### 2. Other Preferred Embodiments

The second to sixteenth embodiments of the present invention are described with reference to Figs. 12 to 36. The second preferred embodiment shows another preferred embodiment of the belt-shaped member 10. The third to the sixth preferred embodiments show other preferred embodiments having the cover member 15. The seventh preferred embodiment shows another preferred embodiment with respect to the position the belt-shaped member 10 is disposed. The ninth preferred embodiment shows another preferred embodiment where the engaging portion 9r is not provided in the belt-shaped member 10. The ninth preferred embodiment shows another embodiment where the direction of expansion and contraction of the belt-shaped member 10 is different. The tenth and eleventh preferred embodiments show other preferred embodiments with respect to a guide element of the belt-shaped member 10. The twelfth to fourteenth preferred embodiments show other preferred embodiments with respect to the implication of the liquid permeable region 21 of the sanitary napkin body 5. The fifteenth and sixteenth preferred embodiments show other preferred embodiments with respect to the arrangement of the belt-shaped member 10.

In the following description, the same reference numerals have been retained for similar portions which are identical to those described in the first preferred embodiment, with the description thereof omitted.

### 2-1. Second Preferred Embodiment

A sanitary napkin 1B in the second preferred embodiment of the present invention is described with reference to Figs. 12 to 16. As shown in Figs. 12 to 16, the sanitary napkin 1B is different from the first preferred embodiment in a belt-shaped member 10B. Specifically, in the sanitary napkin 1B, an inflexible sheet member is used as the belt-shaped member 10B. More specifically, as shown in Figs. 14 to 15C, the sanitary napkin 1B is arranged so that the belt-shaped member 10B made up of the sanitary napkin body 5 and an inflexible sheet is connected at the connecting portion 8 on the non-skin contact surface. As the inflexible sheet, for example, a nonwoven fabric or a film sheet can be used, and it may be a member having a strength by which the belt-shaped member 10B can withstand the force exerted when pulling up. The inflexible sheet can be in the shape of a belt, a string, or the like, and the belt-shape is preferred. Thus, the sanitary napkin 1B can employ the inflexible material as the belt-shaped member 10B. Alternatively, a plurality of base material sheets of different strengths may be used.

As shown in Fig. 16, a belt-shaped member 10B' in the second preferred embodiment may have a folding portion 17 of a predetermined amount. Having the folding portion 17 enables the belt-shaped member 10B' to be extended. Additionally, arranging the belt-shaped member 10B' in the folded state enables the belt-shaped member 10B' to have a predetermined length and to be accommodated in a compact form.

For example, the predetermined length is preferably in the range of 30% to 300% of the length in the longitudinal direction LD (MD) of the sanitary napkin body, more preferably in the range of 70% to 150%. The predetermined amount of the folding portion 17 includes the length that permits folding back for maintaining the predetermined length of the belt-shaped member 10B.

### 2-2. Third to Sixth Preferred Embodiments

Sanitary napkins 1C, 1D, 1E, and 1F in the third to sixth preferred embodiments of the present invention are described with reference to Figs. 17 to 24B. As shown in Figs. 17 to 24B, the sanitary napkins 1C to 1F are different from the first preferred embodiment in having a cover member 15.

Referring to Figs. 17 to 19C, the sanitary napkin 1C in the third preferred embodiment has the cover member 15 covering at least a portion of the belt-shaped member 10 on the non-skin contact surface of a sanitary napkin body 5C. Specifically, the cover member 15 covers at least a portion in the longitudinal direction LD (MD) and the entire width direction WD (CD) of the belt-shaped member 10. The belt-shaped member 10 is disposed so as to be slidable in between the sanitary napkin body 5C and the cover member 15.

In the third preferred embodiment, the cover member 15 and the sanitary napkin body 5C can be connected to each other on both side portions of the sanitary napkin 1C and on the non-skin contact surface at the front end in the front region (F). More specifically, these can be connected to each other on both side portions of the back sheet portion 3 and at the front end in the front region (F).

Alternatively, the cover member 15 may be connected to the back sheet portion 3 at both side portions of the sanitary napkin 1C and at the front end in the front region (F), in the state where the cover member 15 is extended from both side portions of the sanitary napkin 1C and from the front end in the front region (F).

The length of the cover member 15 in the longitudinal direction LD (MD) is preferably, for example, in the range of 10% to 100%, more preferably in the range of 50% to 90% of the length of the sanitary napkin body 5C in the longitudinal direction LD (MD). This is because when it is below 10%, the degree of freedom between the sanitary napkin body 5C and the belt-shaped member 10 is too large, involving a risk of slipping when the sanitary napkin 1C is being put on. This is also because when it is above 100%, the cover member 15 becomes an obstacle in the operation of extending and engaging the belt-shaped member 10 with the underwear 50.

The length of the cover member 15 in the width direction WD (CD) is preferably, for example, in the range of 30% to 150%, and more preferably in the range of 50% to 110% of the length of the sanitary napkin body 5C in the width direction WD (CD). This is because when it is below 30%, the sanitary napkin body 5C cannot make sufficiently close contact with the human body. This is also because when it is above 150%, the area to be contacted with the femoral region is excessive, involving the risk of skin irritation due to frictional contact or the like.

The length of the cover member 15 in the width direction WD (CD) is preferably, for example, in the range of 100% to 200%, and more preferably in the range of 105% to 150% of the length of the belt-shaped member 10 in the width direction WD (CD). This is because when it is below 100%, the belt-shaped member 10 cannot be extended smoothly. This is also because when it is above 200%, the degree of freedom of the belt-shaped member 10 in the width direction WD (CD) is too large, involving the likelihood of misalignment between the central axis of the sanitary napkin body 5C and the central axis of the belt-shaped member 10.

The cover member 15 may be formed of a different material from the construction materials of the sanitary napkin body 5C. Alternatively, any one of these construction materials may also be used for forming the cover member 15. In this case, a common construction material may be elongated so as to continuously form the cover member 15. For example, the top sheet portion 2 or the back sheet 3 may be used successively. In this case, the respective sheets disposed continuously may be overlapped and connected to each other on the non-skin contact surface. More specifically, 50% or more of the length of the sanitary napkin body 5C in the width direction WD (CD) may be continuous and the respective extended sheets may be folded back for lamination and connection, at the substantially central portion in the width direction on the non-skin contact surface of the sanitary napkin body 5C.

As shown in Fig. 20, in a sanitary napkin 1D in the fourth preferred embodiment, a cover member 15D is disposed in the rear region (R) of a sanitary napkin body 5D. Both side portions of the cover member 15D are folded inside the sanitary napkin body 5D, and connected to the back sheet portion 3 (refer to Fig. 21A). This arrangement allows for a large frontage between the back sheet portion 3 and the cover member 15D, thereby facilitating, for example, expansion of the belt-shaped member 10.

As shown in Fig. 21B, in the cover member 15D, an elastic member 16 may be arranged at each of the folded portions of the cover member 15D on both side portions in the width direction WD (CD) of the sanitary napkin body 5D. This arrangement prevents the sanitary napkin body 5D or the belt-shaped member 10 having a tendency to become kinked, by way of the force to return to the original form being able to be generated by the elastic force of the elastic members 16, and thereby preventing the kink of the sanitary napkin body 5D or the belt-shaped member 10.

As shown in Figs. 22A and 22B, in a sanitary napkin 1E in the fifth preferred embodiment, a cover member 15E is arranged so as to cover the engaging portion 9r of the belt-shaped member 10. In this case, when the belt-shaped member 10 is in an inflexible state, the grip portion 6 of the belt-shaped member 10 is extended from the outer edge in the rear region (R) of a sanitary napkin body 5E in the longitudinal direction LD (MD), and the engaging portion 9r is covered with a cover member 15E. By covering the engaging portion 9r with the cover member 15E, sticking the engaging portion 9r, for example, to any unintentional location before extending the belt-shaped member 10 is avoidable. Additionally, the extension of the grip portion 6 enables, for example, the wearer to easily pinch the grip portion 6, thereby increasing operability.

Alternatively, a silicon resin or the like may be coated on the surface of the cover member 15E. This is because the coating of the silicon resin or the like prevents the engaging portion 9r from sticking to the cover member 15E. Alternatively, the surface of the engaging portion 9r may be covered with a peelable sheet or the like.

A plurality of the cover member 15E may be provided, and these are preferably arranged at least in the vicinity of the rear region (R) of the sanitary napkin body 5E. This is because this arrangement can prevent the belt-shaped member 10 from slipping from the sanitary napkin body 5E when the sanitary napkin is being put on.

As shown in Fig. 22B, the elastic member 16 may be arranged at each of the folded portions of the cover member 15E on both side portions in the width direction WD (CD) of the sanitary napkin body 5E.

As shown in Figs. 23 to 24B, in a sanitary napkin 1F in the sixth preferred embodiment, engaging portions 91R and 91L to be engaged with the underwear 50 may be arranged on both sides in the substantially central portion of a cover member 15F in the width direction WD (CD). With this arrangement, the underwear 50 can also be lifted to the human body by the tension of the belt-shaped member 10. This prevents, for example, dislocation between the sanitary napkin 1F and the underwear 50.

A plurality of the engaging portions 91R and 91L, for example 2, may be arranged on each of both sides in the substantially central portion of the cover member 15F in the width direction WD (CD). In an alternative, these may be arranged throughout the width direction WD (CD) in the substantially central portion of the cover member 15F. In another alternative, these may be arranged in the range of from the substantially central portion to the rear region (R) of the cover member 15F. In still another alternative, these may be arranged throughout the entire cover member 15F.

### 2-3. Seventh Preferred Embodiment

A sanitary napkin 1G in the seventh preferred embodiment of the present invention is described with reference to Figs. 25 to 26B. As shown in Figs. 25 to 26B, the sanitary napkin 1G is different from the first preferred embodiment in the position where the belt-shaped member 10 is provided.

More specifically, in the sanitary napkin 1G, a tissue 72 is disposed between the top sheet portion 2 and the absorbent body portion 4. The belt-shaped member 10 is disposed between the tissue 72 and the absorbent body portion 4. In this case, the top sheet portion 2 and the back sheet portion 3 are connected to each other on both side portions of the sanitary napkin 1G and at the front end in the front region (F) (refer to Fig. 26A). The belt-shaped member 10 extends from an opening that is a non-connecting portion in the rear region (R) of the sanitary napkin 1G. Alternatively, the belt-shaped member 10 may be disposed between the top sheet portion 2 and the tissue 72.

In the seventh preferred embodiment, the base material sheets 11 and 13 of the belt-shaped member 10 are preferably hydrophilic and liquid permeable. It is therefore possible to effectively facilitate mobility of excrement such as menstrual blood from the top sheet portion 2 to the absorbent body portion 4. Alternatively, the belt-shaped member 10 may be disposed between the absorbent body portion 4 and the back sheet portion 3, as shown in Fig. 26B. When an absorbent body portion (not shown) is made up of a plurality of layers, the belt-shaped member 10 may be interposed between the layers of the absorbent body portion.

### 2-4. Eighth Preferred Embodiment

A sanitary napkin 1H in the eighth preferred embodiment of the present invention is described with reference to Figs. 27A and 27B. As shown in Figs. 27A and 27B, the sanitary napkin 1H is different from the first preferred embodiment in the direction in which the belt-shaped member 10 expands and contracts. More specifically, in the sanitary napkin 1H, the belt-shaped member 10 expands and contracts in the direction of the front region (F).

In this case, there are the following manners of application. In a first example, after the underwear 50 is pulled down to the vicinity of the wearer's knees, the engaging material at the engaging portion 9r in the rear region (R) of a sanitary napkin body 5H is engaged and fixed to the underwear 50. Subsequently, the engaging portion 9r is pressed by one hand, and the grip portion 6 is gripped by the other hand. In this state, the belt-shaped member 10 is extended to the front region (F), and the engaging material of the engaging portion 9f is engaged and fixed to a predetermined position of the underwear 50. The underwear 50 is put on when the attachment of the sanitary napkin 1H to the underwear 50 is completed.

In a second example, after the underwear 50 is pulled down to the vicinity of the wearer's knees, the engaging material at the engaging portion 9r in the rear region (R) of a sanitary napkin body 5H is engaged and fixed to the underwear 50. In this state, the underwear 50 is put on. Next, from the front region (F), a hand is put in between the underwear 50 and the human body, and the grip portion 6 is gripped and the belt-shaped member 10 is pulled to the front region (F) in order to fix the engaging material of the engaging portion 9f to the underwear 50. Thus, in the sanitary napkin 1H, the belt-shaped member 10 may expand and contract to the front region (F).

### 2-5. Ninth Preferred Embodiment

A sanitary napkin 1I in the eighth preferred embodiment of the present invention is described with reference to Figs. 28A and 28B. As shown in Figs. 28A and 28B, the sanitary napkin 1I is different from the first preferred embodiment in that the engaging portion 9r to be engaged with the underwear 50 is not provided in a belt-shaped member 10I in the rear region (R). That is, the sanitary napkin 1I has no engaging portion 9r engaged with the underwear 50 in the rear region (R).

In this case, there is the following manner of application. After pulling down the underwear 50 to the wearer's knees, engaging portions 9f, 91R, and 91L are fixed to the underwear 50 in the front region (F) and at both side portions in a sanitary napkin body 5I, respectively, and the underwear 50 is then put on. Subsequently, from the back of the human body, a hand is put in between the underwear 50 and the human body, the grip portion 6 is gripped, and the belt-shaped member 10I is pulled along the curvature of the human body to the rear region (R) so that it is inserted in the groove in the vicinity of the excretory part, namely the groove in the vicinity of the gluteal region. Thus, the engaging portion 9r to be engaged with the underwear 50 in the rear region (R) can be omitted from the sanitary napkin body 5I.

### 2-6. Tenth and Eleventh Preferred Embodiments

Sanitary napkin 1J and 1K in the tenth and eleventh preferred embodiments of the present invention are described with reference to Figs. 29 to 30B, respectively. As shown in Figs. 29 to 30B, the sanitary napkins 1J and 1K are different from the first preferred embodiment in having guide elements 73a and 73b, respectively. More specifically, in the sanitary napkin 1J and 1K in the tenth and eleventh preferred embodiments, the grip portions 6J and 6K may be provided with the guide elements 73a and 73b that indicate the directions of extension of the belt-shaped members 10J and 10K, respectively.

The guide elements 73a and 73b can be attained by indication sign sheets such as arrows, symbols, illustrations, characters, colors, or color gradation, or attained by feel such as embossing. Alternatively, the guide elements 73a and 73b may be formed with the pattern treatment of a hot melt adhesive to the skin-contact surface or the non-skin contact surface of the grip portions 6J and 6K, respectively. When the guide elements 73a and 73b are formed by color pattern treatment of a hot melt adhesive, the base material sheets 11 and 13 in the grip portions 6J and 6K can be stuck to each other at the same time. In an alternative, an indication sign sheet with a guide sign or the like printed thereon may be interposed between the base material sheets 11 and 13. In another alternative, a guide sign or the like may be directly printed on the base material sheets 11 and 13.

In the tenth preferred embodiment, the grip portion 6J extends from the outer edge in the rear region (R) of the sanitary napkin body 5J in the longitudinal direction LD (MD), and the skin-contact surface of the grip portion 6J is exposed. Hence, by adding the guide element 73a to the grip portion 6J, for example, the wearer can easily find the grip portion 6J, thus increasing operability. In an alternative, the grip portion 6J may have a different color from the surrounding color of the belt-shaped member 10J. In other alternative, the guide element 73a may be, for example, a symbol, an illustration, or a character. In a still another alternative, the grip portion 6J may be in the shape of a triangle, a circle, an ellipse, or corrugation.

In the eleventh preferred embodiment, as shown in Figs. 30A and 30B, the grip portion 6K may have the guide element 73b for implying the position of the engaging portion 9r to be engaged with the underwear 50. For example, Fig. 30B illustrates the sanitary napkin 1K in which a colored sheet is arranged on the surface where the engaging material is provided at the engaging portion 9r to be engaged with the underwear 50. The guide element 73b for implying the position of the engaging portion 9r may be a colored sheet, as well as embossing, printing, colored hot melt adhesive, or the like. Alternatively, the base material sheets 11 and 13 of the belt-shaped member 10K may be formed of a sheet of high transparency so that the engaging portion 9r disposed on the back sheet portion 3 is made visible.

### 2-7. Twelfth to Fourteenth Preferred Embodiments

Sanitary napkins 1L, 1M, and 1N in the twelfth to the fourteenth preferred embodiments of the present invention are described with reference to Figs. 31A to 33, respectively. As shown in Figs. 31A to 33, the sanitary napkins 1L, 1M, and 1N are different from the first preferred embodiment in having a guide element. More specifically, the sanitary napkins 1L, 1M, and 1N may have a guide element for implying the liquid permeable region 21 of the sanitary napkin 1.

In the twelfth preferred embodiment, as shown in Figs. 31A and 31B, for example, wings W1 and W2 may be disposed on both side portions of the sanitary napkin 1L, in order to imply that the liquid permeable region 21 is present at the substantially central portion in the width direction WD (CD) of the wings W1 and W2.

In the thirteenth preferred embodiment, as shown in Fig. 32, guide elements 70 having a different color may be disposed, for example, at a predetermined position on both side portions of the sanitary napkin 1M, respectively, in order to imply that the liquid permeable region 21 is present at the substantially central portion in the width direction WD (CD) of the guide elements 70.

As a means for implying the liquid permeable region 21, the position corresponding to the wearer's excretory part, as well as the positions in front and behind thereof may be specified.

In the fourteenth preferred embodiment, as shown in Fig. 33, for example, a guide element 71 for implying the rear region (R) may be provided at a predetermined position of the grip portion 6N of a belt-shaped member 10N. The guide element 71 may be formed by embossing, colored hot melt, printing, or the like. Alternatively, a different color from the surrounding color, as well as color gradation, a symbol, an illustration, a character, and a graphic symbol, may be used to imply the rear region (R).

### 2-8. Fifteenth and Sixteenth Preferred Embodiments

Sanitary napkins 1P and 1Q in the fifteenth and sixteenth preferred embodiments of the present invention are described with reference to Fig. 34 to 36. As shown in Figs. 34 to 36, the sanitary napkins 1P and 1Q are different from the first preferred embodiment in the arrangement of the belt-shaped member 10. More specifically, in the sanitary napkin 1P of the fifteenth preferred embodiment, a pair of belt-shaped members 10P extending in the longitudinal direction LD (MD) may be arranged in a sanitary napkin body 5P in the width direction WD (CD). Alternatively, a plurality of the belt-shaped members 10P may be arranged there.

Thus, the pair of the belt-shaped members 10P are provided on the non-skin contact surface of the sanitary napkin body 5P so that they are connected to the back sheet portion 3 in the front region (F) of the sanitary napkin body 5P, specifically at connecting portions 8a and 8b disposed at the substantially central portion. This enables, for example, the respective belt-shaped members 10P to smoothly follow the movement of the buttocks, which move independently when walking.

In the sixteenth preferred embodiment as shown in Figs. 35 and 36, in the sanitary napkin 1Q, two belt-shaped members 18 and 19 may be arranged so as to stack upon one another in the thickness direction. In this case, as shown in Fig. 36, the sanitary napkin 1Q has the first belt-shaped member 18 and the second belt-shaped member 19. The first belt-shaped member 18 is connected to the back sheet portion 3 at the substantially central portion. The second belt-shaped member 19 is connected to the back sheet portion 3 of a sanitary napkin body 5Q at the rear end in the front region (F). The first and second belt-shaped members 18 and 19, respectively, are covered with a cover member 15Q in the back sheet portion 3 and engaged by an engaging material at the end portion in the rear region (R) of the sanitary napkin body 5Q. For example, the first belt-shaped member 18 has a length of about 50% of the length of the sanitary napkin body 5Q, and the second belt-shaped member 19 has a length of about 95% of the length of the sanitary napkin body 5Q.

Thus, by stacking the first and second belt-shaped members 18 and 19 upon one another, for example, the force induced in the range of from the excretory part to the anus in the rear region (R) can be further increased to improve adhesion.

In an alternative other than the aforementioned embodiments, the belt-shaped member may be used as an auxiliary pad by applying it to the skin-contact surface of another sanitary napkin body. In this case, the belt-shaped member is preferably shorter in length in the width direction WD (CD) and in the longitudinal direction LD (MD) than the sanitary napkin body to be used together. In another alternative, the auxiliary pad formed of the belt-shaped member may be entirely formed of a liquid permeable material, or a liquid impermeable material partially provided with liquid-communication pores, in order to facilitate movement of menstrual blood from this auxiliary pad to the sanitary napkin body.

### 3. Materials Constituting Sanitary Napkin

Components of the sanitary napkin are described hereinafter.

### 3-1. Sanitary Napkin Body

### 3-1-1. Top Sheet Portion

At the time of use, the top sheet portion 2 is disposed on the human body side and also brought into contact with the excretory part. The top sheet portion 2 may be entirely or partly liquid permeable, and it may be made of a sheet member or a plurality of sheet members adhered to each other.

As the top sheet portion 2, a woven fabric, a nonwoven fabric, or a sheet material having liquid permeability such as a porous plastic sheet can be used. Examples of the woven fabric or the nonwoven fabric are natural fibers and chemical fibers. More specifically, as the natural fiber, there are celluloses such as pulverized pulp and cotton. As the chemical fiber, there are regenerated celluloses such as rayon and fibril rayon, semi-synthetic celluloses such as acetate and triacetate, thermoplastic hydrophobic chemical fiber, and thermoplastic hydrophobic chemical fiber subjected to hydrophilization. Examples of the thermoplastic hydrophobic chemical fiber are single fibers such as polyethylene (PE), polypropylene (PP), polyethylene terephthalate (PET), fiber obtained by grafting copolymerization of polyethylene (PE) and polypropylene (PP), and composite fibers such as sheath-core structure.

When a nonwoven fabric is used, dry or wet web foaming such as a card process, spun bond process, melt blown process, and air-laid process can be used. The web foaming may be a combination of the dry and wet types. As a method of bonding, there are thermal bonding, needle punch, chemical boding, and the like, but not limited to these methods. Alternatively, spun lace formed in the shape of a sheet by a spun lace process may be used.

As the porous plastic sheet, there are, for example, a porous sheet made of polyethylene (PE), polypropylene (PP), or polyethylene terephthalate (PET), and porous foaming member.

Preferably, the porous plastic sheet is used in the clouded state by adding filler such as titanium oxide or calcium carbonate in the range of 0.5% to 10%. Alternatively, a film may be obtained by providing pores in a thermoplastic resin film by perforation, heat embossing finish, or cutting. The porous film may be combined with the nonwoven fabric to form a composite sheet.

### 3-1-2. Absorbent Body Portion

It is necessary for the absorbent body portion 4 to be able to retain and absorb excrement such as menstrual blood. Preferably, it is difficult for the absorbent body portion 4 to become bulky and lose its shape, and has less chemical irritation. There are celluloses such as pulverized pulp and cotton, regenerated celluloses such as rayon and fibril rayon, semi-synthetic celluloses such as acetate and triacetate, particulate polymer, fibrous polymer, thermoplastic hydrophobic chemical fiber, thermoplastic hydrophobic chemical fiber subjected to hydrophilization, and air-laid pulp subjected to a chemical bonding process. These can be used singly or mixed together.

Although no special limitation is imposed on the method of forming these materials in the absorbent body portion 4, an air-laid process, melt blown process, spun lace process, or paper making process can be used for sheet forming.

As the absorbent body portion 4, cellulose foam or continuous foam of synthetic resin can also be used. Alternatively, the sheet-shaped material or foam may be pulverized, and then formed into an absorbent body.

Among others, a preferred sheet-shaped absorbent body has a fiber basis weight of 100 g/m² to 2000 g/m², and a bulk of 1 mm to 50 mm, which can be obtained by mixing pulp in the range of 80% to 100%, and particulate polymer in the range of 20% or less, and then coating with tissue, followed by an embossing finish. The embossing finish is for preventing the absorbent body from losing its shape. The embossing area ratio is preferably in the range of 10% to 100%, and more preferably 30% to 80%.

Other examples of the material of the absorbent body portion 4 are an absorption sheet and a polymer sheet, each preferably having a thickness of 0.3 mm to 5.0 mm. The absorption sheet and the polymer sheet may be those used for absorbent articles such as sanitary napkins.

Examples of the absorption sheet are an absorption paper, a nonwoven fiber, and a pulp sheet obtained by forming fiber in a sheet with binder. Examples of the polymer sheet are a pulverized pulp and a sheet obtained by mixing particulate polymer in a fiber, and forming the mixture in a sheet. In the sheet thus obtained, the particulate polymer may be dispersed in the shape of a layer or in three dimensions.

The material forming the absorption sheet and the fiber used in the polymer sheet are preferably any one of cellulose fibers such as wood pulp, regenerated celluloses such as rayon and cupra, hydrophilic synthetic fibers such as a polyvinyl alcohol fiber and a polyacrylonitrile fiber, as well as polyethylene, polypropylene, polyethylene terephthalate, a polyethylene/polypropylene composite fiber, a polyethylene/polyethylene terephthalate composite fiber, whose fiber surface is hydrophilized by a surface active agent. More preferred is the cellulose fiber in consideration of hydrophilic properties.

A preferred particulate polymer used in the polymer sheet are ones capable of absorbing and retaining liquid whose weight is 20 times its own weight, and capable of being gelatinized. There are, for example, starch, cross-linked carboxymethyl cellulose, polyacrylic acid and a salt thereof, and polyacrylic acid salt graft copolymer.

### 3-1-3. Back Sheet Portion

As the back sheet portion 3, a thermoplastic film composed mainly of polyethylene (PE) or polypropylene (PP), a permeable resin film, one obtained by connecting a permeable resin film to a nonwoven fiber such as spun bond or spun lace, a multilayer of SMS (spun bond/melt blown/spun bond), or the like can be used. Preferred is a film that is composed mainly of low-density polyethylene (LDPE) resin and has a fiber basis weight in the range of 15 g/m² to 30 g/m². This film has flexibility and does not damage fit feeling.

When a liquid impermeable sheet is used in the belt-shaped member, the back sheet portion 3 may be the same liquid permeable sheet as the top sheet portion 2.

### 3-1-4. Connecting Portion

The top sheet portion 2 and the absorbent body portion 4 can be connected to each other by adhering them with a hot melt adhesive, respectively. The top sheet portino 2 and the back sheet 3 can be connected so that these are adhered to each other by a connecting portion formed by a hot melt adhesive and hot pressing. As a whole, the surfaces between the adjacent sheets are adhered by the hot melt adhesive, and the ends of the sheets are connected by the connecting portion formed by the hot pressing process. The adhesion is not limited to that by the hot melt adhesive. For example, the heat embossing finish and ultrasonic wave may be used singly or in combination.

As the coating pattern when the hot melt adhesive is used for connection, there are, for example, spiral coating, control seam coating, coater coating, curtain coating, and summit gun coating. The basis weight of adhesive in the hot melt adhesion is preferably 1 g/m² to 30 g/m², more preferably 3 g/m² to 10 g/m². The diameter adhesive coated in a pattern linearly is preferably from 30 µm to 300 µm.

### 3-2. Belt-Shaped Member

### 3-2-1. Base Material Sheet

As the base material sheets 11 and 13, for example, a spun/bond nonwoven fabric composed mainly of polypropylene (PP) can be used. In this case, the fiber basis weight is preferably from 15 g/m² to 25 g/m². The fineness is preferably from 1.5 dtex to 2.5 dtex.

When the back sheet portion 3 is a liquid impermeable sheet, for example, each of the base material sheets 11 and 13 of the belt-shaped member 10 can employ the liquid permeable sheet as exemplified as the top sheet portion 2. The base material sheets 11 and 13 are required to be a nonwoven fabric having a thin thickness, such as a spun bond nonwoven fabric, a point bond nonwoven fabric, or a spun lace nonwoven fabric. This is because the nonwoven fabric having a thin thickness can enhance the smoothness in the expanded and contracted states of the belt-shaped member. When the concave and convex shapes are applied by the corrugated embossing finish as shown in the first preferred embodiment, it is preferable to use a spun bond nonwoven fabric composed of a continuous fabric, in order to suppress the nonwoven fabric from being broken during the liquid embossing finish.

When the back sheet portion 3 is a liquid permeable sheet, each of the base material sheets 11 and 13 of the belt-shaped member 10 can employ the liquid impermeable sheet as exemplified above as the back sheet portion 3. The liquid impermeable sheet may be disposed only on the skin-contact surface of the elastic member, or disposed on both of the skin-contact surface and the non-skin contact surface.

### 3-2-2. Elastic Member

The elastic member 12 can employ, for example, an elastic string of natural rubber or polyurethane. More specifically, foams of elastomer component or polyethylene foam can be used singly, or alternatively, one obtained by forming a mixture of these in the shape of a belt or a sheet can be used. The fineness of the elastic string is preferably from 350 dtex to 450 dtex. For example, the number of the elastic string is from seven to nine.

Examples of the elastomer component are thermoplastic elastomer of polyester, urethane, olefin, styrene, or polyamide, low density polyethylene using metallocene catalyst, and ethylene-α-olefin copolymer. These can be used singly, or a plurality of types thereof may be blended.

Examples of the polyester elastomer are those whose hard segment is an aromatic polyester and soft segment is a non-crystalline polyether or aliphatic polyester.

Examples of the urethane elastomer are polyurethane composed of polyester, low molecular weight glycol, and methylene bisphenyl isocyanate, in which polyisocyanate is added to polylactone ester polyol and polymerized in the presence of short chain polyol.

Examples of the olefin elastomer are ethylene-α-olefin random copolymer, and one in which diene is copolymerized as a third composition.

Examples of the styrene elastomer are block copolymers such as SEBS, SIS, SEPS, and SBS.

Examples of the polyamide elastomer are those whose hard segment is nylon and soft segment is polyester or polyol.

In order to stabilize the formation of the elastic member, the constitutive polymer of the elastomer composition may contain, for example, high density polyethylene, low density polyethylene, or linear low density polyethylene. It may further contain a blocking inhibitor, ultraviolet absorbing agent, thickening and branching agent, flatting agent, coloring agent, and other various improvers. Among others, the polyurethane elastic string is preferred due to less influence by heat or strain.

### 3-2-3. Absorbent body Portion

When an absorbent body portion 100 is interposed between the base material sheets 11 and 13 of the belt-shaped member 10, the same material as the absorbent body portion 4 in the sanitary napkin body 5 can be used as the constitutive material of the absorbent body portion 100.

When a material having absorptivity is used in the base material sheets 11 and 13 of the belt-shaped member 10, for example, it is possible to use any woven fibers and nonwoven fibers which are sheet-shaped materials and natural fibers or chemical fibers. Examples of the natural fiber are celluloses such as pulverized pulp and cotton. Examples of the chemical fiber are regenerated celluloses such as rayon and fibril rayon, semi-synthetic celluloses such as acetate and triacetate, thermoplastic hydrophobic chemical fiber, and thermoplastic hydrophobic chemical fiber subjected to hydrophilization.

When the nonwoven fabric is used, web foaming of dry type (card process, spun bond process, melt blown process, air-laid process, or the like) or of wet type may be performed. Alternatively, these may be combined. Examples of a method of bonding are, without being limited to these methods, thermal bonding, needle punch, chemical boding, and the like. Alternatively, a spun lace formed in the shape of a sheet by spun lace process may be used.

### 3-2-4. Connecting Material

As the connecting material in connection between the base material sheets 11 and 13 and the elastic member 12, or between the base material sheets 11 and 13, heat embossing finish, ultrasonic wave, or hot melt type adhesive can be used singly or in combination. For example, a hot melt adhesive is applied to the base material sheet 11 by a coating method such as spiral coating, coater coating, curtain coater coating, or summit gun coating, and the elastic material 12 is laid thereon, then the base material sheet 13 is laid thereon and adhered together. In order to prevent the elastic member 12 from separating from the base material sheets 11 and 13, the elastic member 12 may be coated in advance by a coating method such as slit coating or control seam coating.

Examples of the hot melt adhesive are pressure sensitive adhesives and thermo-sensitive adhesives each composed mainly of rubbers such as SEBS, SBS, and SIS, or olefins such as linear low-density polyethylene; and water-sensitive adhesives of polyvinyl alcohol, carboxyl methyl cellulose, or gelatin each being composed of water soluble high polymer, or of polyvinyl acetate or polyacrylic acid sodium each being composed of water swelling high polymer. For example, it is preferable to use a heat sensitive adhesive that, even if the aforementioned adhesive effuses, has no tackiness at that point. Specific examples are melt-mixtures of 5% to 25% of SEBS, 40% to 60% of alicyclic saturated hydrocarbon, 1% to 10% of aromatic denaturated terpene, and 15% to 35% of additive.

### 3-2-5. Engaging Material

As the engaging material at the engaging portion 9 for engaging the sanitary napkin body to the underwear 50, for example, a hot melt adhesive can be used. Preferably, the hot melt adhesive has tackiness at an ordinary temperature, such as a pressure-sensitive adhesive. The main component of this adhesive is the same as in the abovementioned connecting material. Specifically examples are melt-mixtures of 15% to 25% of SEBS, 15% to 35% of plasticizer, and 40% to 70% of an adhesive-imparting agent. Furthermore, an oxidation inhibitor and fluorescence inhibitor may be added in the range of 0.1% to 1.0%. The basis weight is from 10 gsm to 200 gsm, and coated uniformly or in a pattern of stripe-shape or dot-shape by coater coating or bead coating. In an alternative, an acrylic adhesive may be used. In another alternative, the engagement may be attained by using a plurality of hook portions that stand on the surface of each tape-shaped portion.

Specifically, the tape-shaped portion can be formed by extrusion molding of a thermoplastic synthetic resin such as polypropylene, followed by cutting and removal of a rib structure portion integrally formed with the tape portion. As the result, a hook portion can be formed on the surface of the tape-shaped portion. In an alternative, a hook portion may be formed by cutting from a side a monofilament loop composed of thermoplastic synthetic resin which is provided on the surface of the tape-shaped portion. In another alternative, the end face of the hook portion may be rounded in order to eliminate the danger of damaging the skin. More specifically, the top of the hook can be rounded with the shape of a die. No special limitation is imposed on the cross-sectional shape of the hook portion, and it may be tapered or of T-shape.

On the other hand, as the engaging portion 9 to be fixed to the wearer's body and not to the underwear 50, when the engaging portion 9 is provided on the skin-contact surface of the belt-shaped member 10, there are, for example, a water-soluble polymer, crosslinking agent, plasticizer, gel adhesive composed of water, and the like. Examples of the water-soluble polymer are gelatin, polyacrylic acid sodium, polyvinyl alcohol, and carboxyl methyl cellulose. Examples of the crosslinking agent are water-soluble metallic salts such as calcium chloride and magnesium sulfate. Examples of the plasticizer are glycerine, wax, and paraffin.

With respect to the pressure-sensitive adhesive and the engaging portion 9, the portion having tackiness is preferably covered with a sheet in which silicon resin has been coated on tissue paper as generally available release paper, or a sheet in which a silicon resin has been coated on a film. This is because the adhesive portion can be protected from debris or release during storage.

## Claims

1. An absorbent article comprising:
an absorbent article body having at least:
a top sheet portion disposed on a first side of the absorbent article having a substantially elongated shape in a thickness direction, at least a portion of the top sheet portion being liquid permeable, and
a liquid retainable absorbent body portion disposed on the a second side of the top sheet portion, the liquid retainable absorbent body portion being disposed on a second side of the absorbent article in the thickness direction; and
a belt-shaped member disposed along a longitudinal direction of the absorbent article body, the belt-shaped member being disposed on the second side of the absorbent article body,
wherein
a first end of the belt-shaped member in the longitudinal direction is connected to the absorbent article body.

2. The absorbent article according to claim 1, wherein at least a portion of the belt-shaped member is formed so as to be extensible in the longitudinal direction.

3. The absorbent article according to claim 1, wherein at least a portion of the belt-shaped member is formed so as to be able to contract in the longitudinal direction.

4. The absorbent article according to any one of claims 1 to 3, further comprising a cover portion disposed at the second side of the absorbent article body, the cover portion covering at least a portion of the belt-shaped member in the longitudinal direction and covering the entire belt-shaped member in a width direction.

5. The absorbent article according to any one of claims 1 to 4, wherein the belt-shaped member has a grip portion on a second end in the longitudinal direction, the grip portion extending from an outer edge of the absorbent article body in the longitudinal direction.

6. The absorbent article according to claim 5, wherein the grip portion has a guide element implying a direction of extension of the belt-shaped member.

7. The absorbent article according to claim 5 or 6, wherein the second end of the belt-shaped member is provided with a predetermined engaging portion.

8. The absorbent article according to any one of claims 5 to 7, wherein the second end of the belt-shaped member is provided with a predetermined absorptive member.

9. The absorbent article according to claim 8, wherein the absorptive member is disposed in a region extending from the outer edge of the absorbent article body in the longitudinal direction when the belt-shaped member expands and contracts.

10. The absorbent article according to claim 8 or 9, wherein the belt-shaped member has a liquid impermeable sheet disposed in a region corresponding to the absorptive member on the opposite side of the absorbent article body.

11. The absorbent article according to any one of claims 1 to 10, wherein a length of the belt-shaped member in the width direction is in a range of at least 30% of a length of the absorbent article body in the width direction.

12. The absorbent article according to any one of claims 1 to 11, wherein the belt-shaped member has a belt-shaped base material sheet and an elastic member.

13. The absorbent article according to any one of claims 1 to 12, wherein at least a portion of the belt-shaped member has a liquid impermeable material.

14. An absorbent article comprising:
an absorbent article body having at least:
a top sheet portion, at least a portion thereof being liquid permeable, the top sheet portion being disposed on a first side of the absorbent article having a substantially elongated shape in a thickness direction, the absorbent article being used by bringing it into contact with a human body in a state of being disposed on an internal surface of a predetermined clothing, and
a liquid retainable absorbent body portion disposed on a second side of the top sheet portion, the absorbent body portion being disposed on a second side of the absorbent article in the thickness direction; and
a belt-shaped member disposed along a longitudinal direction of the absorbent article body, the belt-shaped member being disposed on a second side of the absorbent article body,
wherein
the belt-shaped member can be disposed along the human body by using the belt-shaped member in a state where it is extended and engaged with an engaged object.

15. An absorbent article comprising:
an absorbent article body having at least:
a top sheet portion, at least a portion thereof being liquid permeable, the top sheet portion being disposed on a first side of the absorbent article in a thickness direction, the absorbent article having a first manner of application and a second manner of application, and
a liquid retainable absorbent body portion disposed on a second side of the top sheet portion, the absorbent body portion being disposed on a second side of the absorbent article in the thickness direction; and
a belt-shaped member disposed along a longitudinal direction of the absorbent article body, the belt-shaped member being disposed on the second side of the absorbent article body,
wherein
the first manner of application is for using the absorbent article by engaging the belt-shaped member with an engaged object, and the second manner of application is for using the absorbent article by not engaging the belt-shaped member with the engaged object.

16. The absorbent article according to claim 15, wherein the first manner of application is for arranging the belt-shaped member along the human body in a state where the belt-shaped member is extended and engaged with the engaged object.

17. An absorbent article comprising:
an absorbent article body having at least:
a top sheet portion disposed on a first side of the absorbent article having a substantially elongated shape in a thickness direction, at least a portion of the top sheet portion being liquid permeable, and
a liquid retainable absorbent body portion disposed on a second side of the top sheet portion, the absorbent body portion being disposed on a second side of the absorbent article in the thickness direction; and
a liquid permeable belt-shaped member disposed between the top sheet portion and the absorbent body portion so as to be slidable along a longitudinal direction of the absorbent article body,
wherein
one end of the belt-shaped member in the longitudinal direction is connected to the absorbent article body.

18. The absorbent article according to claim 17, wherein at least a portion of the belt-shaped member is formed so as to be extensible in the longitudinal direction.

19. The absorbent article according to claim 17, wherein at least a portion of the belt-shaped member is formed so as to be flexible in the longitudinal direction.
